# EUROPEAN PATENT APPLICATION

(11) **EP 4 803 612 A1**
(43) Date of publication of application: **09.09.2026**
(21) Application number: 24885871.4
(22) Date of filing: 01.11.2024
(51) Int. Cl.: C12N 5/077, C12N 1/00

(54) **METHOD FOR PRODUCING PLATEABLE HEPATOCYTES AND APPLICATION THEREOF**

(30) Priority: 01.11.2023 JP 2023187513
(71) Applicant: Public University Corporation Nagoya City University, Mizuho-ku Nagoya-shi Aichi 467-8601 (JP)
(72) Inventor: MATSUNAGA Tamihide, Nagoya-shi, Aichi 467-8603 (JP); IWAO Takahiro, Nagoya-shi, Aichi 467-8603 (JP); SHAHIN Raghda, Nagoya-shi, Aichi 467-8603 (JP)
(74) Representative: Weickmann & Weickmann PartmbB
(86) International application number: PCT/JP2024/039079
(87) International publication number: WO 2025/095107

(57) **Abstract**

It is one object of the present invention to obtain plateable hepatocytes from suspension hepatocytes without using feeder cells. The present invention provides a method for producing plateable hepatocytes from suspension hepatocytes, comprising (ii) culturing suspension hepatocytes in the presence of at least 5 types selected from a ROCK inhibitor, a TGFβ receptor inhibitor, a GSK-3β inhibitor, an epidermal growth factor (EGF), a hepatocyte growth factor (HGF), a sphingophospholipid, and a glycerophospholipid.

## Description

### Technical Field

The present invention relates to a technique of improving the adhesive ability of hepatocytes.

### Background Art

The liver is the organ that plays a central role in drug metabolism. Drug metabolism tests are performed using primary cultured cells (primary hepatocytes) obtained from living livers. When hepatocytes obtained from living livers are seeded and cultured, they are known to become either adherent (plateable) cells that firmly adhere to plates, or suspension cells that float without adhering, or only partially adhere even if they adhere. Plateable cells can generally be cultured for 4 to 5 days, and are highly versatile because they can be used for experiments regarding interactions between cells or between cells and matrices or regarding induction of cytochrome P450 (CYP) enzyme, cholestatic toxicity tests involving bile canaliculi formation, and drug transport evaluation using transwell. On the other hand, since suspension cells cannot adhere to plates, or only partially adhere even if they adhere, they cannot be used for CYP induction experiments, cholestatic toxicity tests, or tests using Transwell, although the enzyme activity of CYP, etc. can be measured. Also, since suspension cells die within a few hours, and cannot be cultured for a long period of time, their applications are limited.

Therefore, it is desirable to use adherent primary hepatocytes for various tests. However, it is unknown whether primary hepatocytes obtained from living liver will become plateable cells or suspension cells, until they are seeded and cultured, and the reason for this is not clear, either. Furthermore, plateable cells are more expensive than suspension cells due to their versatility. For these reasons, there is a need for a technique of converting suspension cells to plateable cells.

Patent Document 1 describes a method for preparing seeded human hepatocytes, comprising: (a) applying human hepatocytes onto a surface in the presence of feeder cells, wherein the feeder cells are endothelial cells and fibroblasts; (b) co-culturing the applied hepatocytes with the feeder cells after step (a); and (c) forming one or more hepatocyte clusters on the feeder cells by the co-cultured hepatocytes, so that the feeder cells adhere onto the surface, and at least 85% of the co-cultured hepatocytes are in one or more hepatocyte clusters, thereby preparing seeded human hepatocytes. However, this method requires the use of feeder cells, raising concerns about complicated procedures, a reduction in experimental reproducibility, and the risk of cell contamination. Moreover, when various tests are performed using cells obtained by co-culturing with feeder cells, the function of the feeder cells may directly affect the evaluation of the tests. Furthermore, in the case of using fibroblasts, an increase in the number of fibroblasts and fibroblast detachment can occur, which can impair the accuracy of quantitative tests when using cells obtained through co-culture with fibroblasts. For these reasons, a feeder-free culture method is required.

Furthermore, Patent Document 2 describes a method for culturing primary hepatocytes, comprising a step of culturing one or more hepatocytes in contact with an extracellular matrix (ECM) in the presence of a developing medium containing a basic medium to which one or more Wnt signaling inhibitors, one or more receptor tyrosine kinase ligands, and one or more epithelial phenotype stabilizers have been added. Non-Patent Document 1 describes that cell proliferation and reprogramming can be induced by culturing primary hepatocytes (plateable-type) in a medium containing YAC (Y27632, A83-01 and CHIR99021) using a collagen-coated culture dish. However, these methods do not demonstrate converting of suspension cells to plateable cells. Further, the present Inventors have cultured suspension-type human primary hepatocytes on a type 1 collagen plate in the presence of YAC using a method similar to that described in Non-Patent Document 1, but it has been found that this did not result in converting to plateable cells.

### Prior Art Documents

### Patent Documents

Patent Document 1: U.S. Patent No. 11,535,827
Patent Document 2: International Publication WO2023/076292

### Non-Patent Documents

Non-Patent Document 1: Katsuda T, Matsuzaki J, Yamaguchi T, Yamada Y, Prieto-Vila M, Hosaka K, Takeuchi A, Saito Y, Ochiya T. Generation of human hepatic progenitor cells with regenerative and metabolic capacities from primary hepatocytes. Elife. 2019 Aug 8; 8:e47313. doi: 10.7554/eLife.47313.

### Summary of Invention

### Objects to be Solved by the Invention

It is an object of the present invention to obtain plateable hepatocytes from suspension hepatocytes without using feeder cells.

Moreover, it is another object of the present invention to apply the obtained plateable hepatocytes o various tests regarding drug metabolism, etc.

### Means for Solving the Problems

As a result of intensive studies directed towards achieving the above-described objects, the present inventors have found that plateable hepatocytes can be produced by culturing suspension hepatocytes under predetermined conditions, thereby completing the present invention.

Specifically, the present invention relates to the following.
[1] A method for producing plateable hepatocytes from suspension hepatocytes, comprising (ii) culturing suspension hepatocytes in the presence of at least 5 types selected from a ROCK inhibitor, a TGFβ receptor inhibitor, a GSK-3β inhibitor, an epidermal growth factor (EGF), a hepatocyte growth factor (HGF), a sphingophospholipid, and a glycerophospholipid.
[2] The production method according to [1] above, comprising (i) culturing suspension hepatocytes in the presence of at least 4 types selected from a ROCK inhibitor, EGF, HGF, a sphingophospholipid, and a glycerophospholipid, before the culture of (ii).
[3] The production method according to [1] or [2] above, wherein the culture of (ii) comprises forming spheroids from suspension hepatocytes.
[4] The production method according to any one of [1] to [3] above, wherein the cultures of (i) and (ii) are carried out on a culture surface coated with a basement membrane component.
[5] The production method according to any one of [1] to [4] above, comprising (iii) culturing the cells in the presence of at least 9 types selected from a ROCK inhibitor, a TGFβ receptor inhibitor, a GSK-3β inhibitor, a cAMP signaling activator, EGF, HGF, a sphingophospholipid, a glycerophospholipid, a γ secretase inhibitor, oncostatin M, and dexamethasone, following the culture of (ii).
[6] The production method according to any one of [1] to [5] above, which comprises performing a passage operation after the culture of (ii).
[7] The production method according to any one of [1] to [6] above, which comprises cryopreserving the hepatocytes and thawing them, after the culture of (ii).
[8] The production method according to any one of [1] to [7] above, wherein the sphingophospholipid is sphingosine-1-phosphate or a salt thereof, and the glycerophospholipid is lysophosphatidic acid or a salt thereof.
[9] The production method according to any one of [1] to [8] above, wherein the ROCK inhibitor is Y27632, the TGFβ receptor inhibitor is A83-01, and the GSK-3β inhibitor is CHIR99021.
[10] The production method according to any one of [1] to [9] above, wherein the suspension hepatocytes are suspension primary hepatocytes.
[11] The production method according to any one of [1] to [10] above, wherein the suspension hepatocytes are human suspension primary hepatocytes.
[12] A method for measuring the metabolism of a test substance, comprising the following steps (1) to (3):
   (1) A step of obtaining plateable hepatocytes by the production method according to any one of [1] to [11] above;
   (2) A step of allowing a test substance to come into contact with the plateable hepatocytes obtained in the step (1); and
   (3) A step of measuring the metabolism of the test substance.
[13] A method for inducing a metabolic enzyme to hepatocytes, comprising the following steps (1) and (2):
   (1) A step of obtaining plateable hepatocytes by the production method according to any one of [1] to [11] above; and
   (2) A step of allowing a metabolic enzyme inducer to come into contact with the plateable hepatocytes obtained in the step (1).
[14] The method according to [13] above, which comprises subjecting the plateable hepatocytes obtained in the step (1) to a sandwich culture.
[15] A method for producing hepatocyte spheroids, comprising the following steps(1) and (2):
   (1) A step of obtaining plateable hepatocytes by the production method according to any one of [1] to [11] above; and
   (2) A step of culturing the plateable hepatocytes obtained in the step (1) to form hepatocyte spheroids.
[16] A method for subjecting hepatocytes to a two-dimensional culture on cell culture inserts, comprising the following steps (1) to (3):
   (1) A step of obtaining plateable hepatocytes by the production method according to any one of [1] to [11] above;
   (2) A step of dissociating the plateable hepatocytes obtained by the step (1) into single cells; and
   (3) A step of subjecting the hepatocytes converted into single cells in the step (2) to a two-dimensional culture on cell culture inserts such as Transwell.
[17] The method according to [16] above, wherein the culture in the step (3) is carried out on a culture surface coated with a basement membrane component.
[18] A medium used in the method according to any one of [1] to [11] above, comprising at least 4 types selected from a ROCK inhibitor, EGF, HGF, a sphingophospholipid, and a glycerophospholipid.
[19] A medium used in the method according to any one of [1] to [11] above, comprising at least 5 types selected from a ROCK inhibitor, a TGFβ receptor inhibitor, a GSK-3β inhibitor, EGF, HGF, a sphingophospholipid, and a glycerophospholipid.
[20] A kit, comprising the medium according to [18] above, the medium according to [19] above, and suspension hepatocytes.

### Advantageous Effects of Invention

According to the present invention, plateable hepatocytes can be produced from suspension hepatocytes.

### Brief Description of Drawings

[Figure 1] Figure 1 shows an outline of the experimental protocol of Example 1. Day 0 indicates a day on which the cells were seeded.
[Figure 2] Figure 2 shows morphological images of HPHs (human primary hepatocytes) from Lot 1 before and after medium change at 3 hours after seeding.
[Figure 3] Figure 3 shows morphological images of HPHs from each of the three lots at Day 10 after seeding.
[Figure 4] Figure 4 shows morphological images of HPHs from Lot 1 after the metabolic enzyme induction assay.
[Figure 5] Figure 5 shows the results of the mRNA expression induction assay for each metabolic enzyme in HPHs. Each value is obtained by correcting the expression level of each mRNA by the expression level of the reference gene (HPRT: hypoxanthine-guanine phosphoribosyltransferase). Omep: omeprazole, Rif: rifampicin, PB: phenobarbital, 0 hrs sHPHs: suspension human primary hepatocytes immediately after thawing, 0 hrs plated: plateable human primary hepatocyte immediately after thawing.
[Figure 6] Figure 6 shows an outline of the experimental protocol of Example 2. Day 0 in the lower row corresponds to the time point when the passage operation was performed on Day 8 in the upper row.
[Figure 7] Figure 7 shows morphological images of HPHs from Lot 1 after the metabolic enzyme induction assay at 7 days after the passage operation.
[Figure 8] Figure 8 shows the results of measuring the metabolic activity of CYP3A4 using HPHs from Lot 1 after the metabolic enzyme induction assay at 7 days after the passage operation. Each value is obtained by quantifying 1-OH MDZ (1-hydroxymidazolam), a metabolite of midazolam (a substrate of CYP3A4), in each culture supernatant by LC-MS/MS.
[Figure 9] Figure 9 shows morphological images of cells after re-culturing of HPHs that were cryopreserved after culture.
[Figure 10] Figure 10 shows an outline of the experimental protocol of Example 3. Day 0 in the lower row corresponds to the time point when the passage operation was performed on Day 8 in the upper row.
[Figure 11] Figure 11 shows morphological images of sandwich-cultured HPHs from Lot 1 at 7 days after the passage operation.
[Figure 12] Figure 12 shows the results of the mRNA expression induction assay of each metabolic enzyme in sandwich-cultured HPHs from Lot 1 at 7 days after the passage operation. The graph notation is the same as in Figure 5.
[Figure 13] Figure 13 shows the results of measuring the metabolic activity of CYP3A4 using sandwich-cultured HPHs from Lot 1 after the metabolic enzyme induction assay, at 7 days after the passage operation. The graph notation is the same as in Figure 8.
[Figure 14] Figure 14 shows morphological images of sandwich-cultured HPHs from Lot 2 at 7 days after the passage operation.
[Figure 15] Figure 15 shows the results of the mRNA expression induction assay for each metabolic enzyme in sandwich-cultured HPHs from Lot 2 at 7 days after the passage operation. The graph notation is the same as in Figure 5 and Figure 12.
[Figure 16] Figure 16 shows the results of measuring the metabolic activity of CYP3A4 using sandwich-cultured HPHs from Lot 2 after the metabolic enzyme induction assay, at 7 days after the passage operation. The graph notation is the same as in Figure 8 and Figure 13.
[Figure 17] Figure 17 shows an outline of the experimental protocol of Example 3. Day 0 in the lower row corresponds to the time point when the passage operation was performed on Day 8 in the upper row.
[Figure 18] Figure 18 shows morphological images of three-dimensional spheroids induced from HPHs at 20 days after the passage operation.
[Figure 19] Figure 19 shows morphological images of hepatocytes obtained after performing the metabolic enzyme induction assay using hepatocytes at 18 days after the passage operation, which were induced by two-dimensional development culture on cell culture inserts from three-dimensional spheroids. Figure 19A shows the results for each of media, DMSO, and omeprazole. Figure 19B shows the results for each of phenobarbital (PB), CITCO (6-(4-chlorophenyl)imidazo[2,1-b][1,3]thiazole-5-carbaldehyde O-(3,4-dichlorobenzyl)oxime), and rifampicin.
[Figure 20] Figure 20 shows the results of a metabolic enzyme induction assay performed using cells at 18 days after the passage operation, which were induced by two-dimensional development culture on cell culture inserts from three-dimensional spheroids. The graph notation is the same as in Figure 5, Figure 12, and Figure 15.
[Figure 21] Figure 21 shows the results of immunofluorescence staining performed using cells at 18 days after the passage operation, which were induced by two-dimensional development culture on cell culture inserts from three-dimensional spheroids. A. DAPI: nucleus (blue); BSEP (bile salt export pump; ABCB11) efflux transporter: bile acid efflux transporter (green); E-CAD: E-cadherin (red); and merged image. B. DAPI: nucleus (blue); NTCP influx transporter: sodium taurocholic acid cotransport transporter (green); ZO-1 tight junction: tight junction molecule ZO-1 (red); and merged image.
[Figure 22] Figure 22 shows an outline of the experimental protocol of Example 5. Day 0 in the lower row corresponds to the time point when the passage operation was performed after the cryopreservation on Day 5 of the upper row.
[Figure 23] Figure 23 shows the cell morphology (three-dimensional culture, cryopreservation, and passage) and CDFDA assay results in Example 5. 1. Spheroid preparation by EZSPHERE, 2. Subsequently, suspension culture of spheroids using ultra-low adhesion plates, 3. Bile canaliculi excretion activity by MRP2, 4. Spheroids that were converted into single cells and were then passaged (two-dimensional culture), and 5. Spheroids that were converted into single cells, were frozen, and were then passaged (two-dimensional culture).
[Figure 24] Figure 24 shows the results of the analysis of mRNA expression of each metabolic enzyme and drug transporter in sandwich-cultured HPHs at 3 days after passage operation following two-dimensional development culture from three-dimensional spheroids. Each value is obtained by correcting the expression level of each mRNA by the expression level of the reference gene (HPRT: hypoxanthine-guanine phosphoribosyltransferase). pHPHs were obtained after culturing plateable human primary hepatocytes for 48 hours.
[Figure 25] Figure 25A shows an outline of the experimental protocol of Example 6. Figure 25B shows the results of evaluating the mRNA expression of caspase 3. M2: medium 2, Y: Y27632, L: lysophosphatidic acid, A: A83-01, and CH: CHIR99021.
[Figure 26] Figure 26 shows an outline of the experimental protocol of Example 7.
[Figure 27] Figure 27 shows morphological images of HPHs. L1: Lot 1, L2: Lot 2, Normal protocol: ordinary protocol, -A,CH: A83-01 and CHIR99021 not used, -Y: Y27632 not used, -A: A83-01 not used, and -CH: CHIR99021 not used.
[Figure 28] Figure 28 shows morphological images of HPHs. L1: Lot 1, L2: Lot 2, -HGF: HGF not used, -S: D-erythro-sphingosine-1-phosphate not used, -LPA: Lysophosphatidic acid not used, -vitamin C: vitamin C not used, -EGF: EGF not used, and -FBS: FBS not used.
[Figure 29] Figure 29 shows morphological images of HPHs. L1: Lot 1, L2: Lot 2, - FBS (+B27-Vitamin A): FBS was not used, and B27 used, from which vitamin A was removed. iMatrix 511 (laminin) was used to pre-coat the porous membrane of the cell culture inserts with iMatrix 511 (laminin), instead of Matrigel. Collagen was used to pre-coat the porous membrane of the cell culture inserts with collagen Type I, instead of Matrigel.
[Figure 30] Figure 30 shows the results of evaluating the mRNA expression of each of albumin, CYP3A4, and CYP1A2 in HPHs.
[Figure 31] Figure 31 shows an outline of the experimental protocol of Example 8.
[Figure 32] Figure 32 shows morphological images of HPHs on Day 10. L1: Lot 1.
[Figure 33] Figure 33 shows the results of the mRNA expression induction assay for CYP3A4 in HPHs.
[Figure 34] Figure 34 shows the results of the mRNA expression induction assay for CYP1A2 in HPHs.

### Embodiments of Carrying out the Invention

### < Method for producing plateable hepatocytes >

In one aspect, the present invention relates to a method for producing plateable hepatocytes from suspension hepatocytes. According to this method, plateable hepatocytes can be produced from suspension hepatocytes. Plateable hepatocytes can be useful because they can be cultured for a long period of time and can be used in various evaluation experiments such as drug metabolism and drug transport.

In one aspect, the present production method comprises (ii) culturing suspension hepatocytes in the presence of at least 5 types selected from a ROCK inhibitor, a TGFβ receptor inhibitor, a GSK-3β inhibitor, an epidermal growth factor, a hepatocyte growth factor, a sphingophospholipid, and a glycerophospholipid.

### [Suspension hepatocytes]

In the present invention, suspension hepatocytes refer to hepatocytes that cannot adhere to a culture surface such as a plate, or that, even if they partially possess adhesive ability, have a different morphology from plateable hepatocytes. In one aspect, in the present invention, suspension hepatocytes refer to hepatocytes that cannot adhere to other cells or extracellular matrices, or, even if they partially possess adhesive ability, have a different morphology from plateable hepatocytes.

In one aspect, the hepatocytes in the present invention refer to cells that constitute the liver, such as hepatic parenchymal cells (cells that perform major liver functions such as bile production and drug metabolism), bile duct epithelial cells, hepatic sinusoidal endothelial cells, hepatic stellate cells, and Kupffer cells. In one aspect, the hepatocytes in the present invention are hepatic parenchymal cells.

In addition, the hepatocytes are preferably primary hepatocytes, and more preferably human primary hepatocytes (HPHs). Primary hepatocytes are hepatocytes collected from living bodies of mammals. Also, this method is applicable to hepatocytes differentiated from stem cells collected from living bodies of mammals, as well as other hepatic cells, including hepatocyte-like cells, reconstructed hepatic cells and hepatic cell lines due to their similar functional characteristics.
Human primary hepatocytes are hepatocytes directly collected from humans, or hepatocytes differentiated from stem cells collected from humans as hepatocyte-like cells.

Suspension hepatocytes generally can survive for only about 5 hours. Moreover, suspension hepatocytes are typically difficult to use in drug metabolic enzyme induction experiments, such as those involving cytochrome P450 (CYP) enzymes, cholestatic toxicity tests involving bile canaliculi formation, and drug transport evaluation tests using Transwell.

### [Plateable hepatocytes]

In the present invention, plateable hepatocytes refer to hepatocytes that exhibit high cell adhesive properties to culture surfaces such as plates and show a cobblestone-like morphology in a two-dimensional culture. In one aspect, in the present invention, plateable hepatocytes refer to hepatocytes that exhibit high cell adhesive properties to other cells or extracellular matrices and show a cobblestone-like morphology in a two-dimensional culture.

Plateable hepatocytes are generally known to be culturable for about 4 to 5 days, but plateable hepatocytes obtained by the present invention can be cultured for an even longer period.

The fact that plateable hepatocytes were produced from suspension hepatocytes can be evaluated by one or more of the following: 1) having obtained hepatocytes that can adhere to culture surfaces such as plates, 2) having obtained hepatocytes that can adhere to other cells or extracellular matrices, and 3) changes in cell morphology. Changes in cell morphology are based on a morphological difference between plateable hepatocytes and suspension hepatocytes. When suspension hepatocytes are cultured in an ordinary culture dish, they generally show no morphological change or little morphological change after culture, remaining spherical, similar to their shape immediately after seeding. In contrast, when plateable hepatocytes are cultured in an ordinary culture dish, they are generally flatter (cobblestone-like) than suspension hepatocytes. Therefore, 3) the change in cell morphology may be that the cells become flatter (cobblestone-like) than suspension hepatocytes. It is to be noted that the change in cell morphology can be confirmed using any microscope suitable for cell observation.

Besides, in the present invention, when the expression "to produce plateable hepatocytes" is used, it is not necessary for all cells in a cell population used for culture to be obtained as plateable hepatocytes, but it is adequate if at least some of the cells are obtained as plateable hepatocytes.

Moreover, while a population of plateable hepatocytes may include cells lacking adhesive ability (for example, approximately several percent to 20%), the production method of the present invention can also be used for the purpose of obtaining plateable hepatocytes from such cells lacking adhesive ability.

### (ii) Culture in the presence of at least 5 types selected from a ROCK inhibitor, a TGFβ receptor inhibitor, a GSK-3β inhibitor, an epidermal growth factor, a hepatocyte growth factor, a sphingophospholipid, and a glycerophospholipid

The method for producing plateable hepatocytes of the present invention comprises (ii) culturing suspension hepatocytes in the presence of at least 5 types selected from a ROCK inhibitor, a TGFβ receptor inhibitor, a GSK-3β inhibitor, an epidermal growth factor (EGF), a hepatocyte growth factor (HGF), a sphingophospholipid, and a glycerophospholipid. (ii) "In the presence of at least 5 types selected from a ROCK inhibitor, a TGFβ receptor inhibitor, a GSK-3β inhibitor, EGF, HGF, a sphingophospholipid, and a glycerophospholipid" is synonymous with a culture system comprising at least 5 types selected from a ROCK inhibitor, a TGFβ receptor inhibitor, a GSK-3β inhibitor, EGF, HGF, a sphingophospholipid, and a glycerophospholipid (hereinafter, when the expression "in the presence of (predetermined components)" is used in the present invention, similarly, it also means a culture system comprising (such predetermined components)), In the present invention, the culture system may include a medium, a culture medium, a culture vessel, etc. In one aspect, the expression "to culture in the presence of at least 5 types selected from a ROCK inhibitor, a TGFβ receptor inhibitor, a GSK-3β inhibitor, EGF, HGF, a sphingophospholipid, and a glycerophospholipid" means to culture in a medium comprising at least 5 types selected from a ROCK inhibitor, a TGFβ receptor inhibitor, a GSK-3β inhibitor, EGF, HGF, a sphingophospholipid, and a glycerophospholipid. The medium is not particularly limited, as long as it can be generally used in the culture of hepatocytes. The medium may be, for example, Dulbecco's Modified Eagle Medium (DMEM).

In the present invention, when the concentration of a specific component is specified in a culture system or a medium, it means that it is not necessary to maintain that concentration throughout the culture period, but it means that the concentration should be maintained at the initiation of the culture.

### (ROCK inhibitor)

Any ROCK (Rho-associated protein kinase: Rho kinase) inhibitor that has an action to inhibit Rho kinase can be used, and Y27632 is preferable. The concentration of the ROCK inhibitor (in the case of Y27632) added to the culture system is, for example, 0.1 to 50 µM, preferably 0.5 to 30 µM, and more preferably 1 to 20 µM. It is to be noted that when a ROCK inhibitor different from Y27632 is used, the concentration added to the culture system can be set by a person skilled in the art in accordance with the above-described concentration range, taking into consideration the differences between the characteristics of the ROCK inhibitor used and those of Y27632 (especially, activity). Furthermore, whether or not the set concentration range is appropriate can be confirmed by preliminary experiments.

### (TGFβ receptor inhibitor)

As TGFβ receptor inhibitors, preferably, those that exhibits an inhibitory activity against one or more of the TGFβ receptors ALK4, ALK5, and ALK7 can be used, and examples of such TGFβ receptor inhibitors may include A83-01, SB431542, SB-505124, SB525334, D4476, ALK5 inhibitor, LY2157299, LY364947, GW788388, and RepSox, with A83-01 being particularly preferred. Alternatively, a peptide compound having a TGFβ inhibitory action (an alternative peptide compound) may be used instead of the TGFβ receptor inhibitor. The peptide compound having a TGFβ inhibitory action may be a TGFβ inhibitor peptide, i.e., one that binds to TGFβ and inhibits the binding of TGFβ and the TGFβ receptor. Otherwise, the peptide compound having a TGFβ inhibitory action may also be a TGFβ receptor inhibitor peptide, i.e., one that binds to a TGFβ receptor and inhibits the binding of TGFβ and the TGFβ receptor. An example of the TGFβ inhibitor peptide may be PG-002 (PeptiGrowth), a TGFβ1 inhibitor peptide. The concentration of the TGFβ receptor inhibitor (in the case of A83-01) added to the culture system is, for example, 0.05 to 10 µM, preferably 0.1 to 5 µM, and more preferably 0.5 µM to 3 µM. When a compound different from A83-01 is used, the concentration thereof can be set by those skilled in the art in accordance with to the above-described concentration range, taking into consideration the differences between the characteristics of the compound used and those of A83-01 (especially, differences in activity). Moreover, whether or not the set concentration range is appropriate can be confirmed by preliminary experiments.

### (GSK-3β inhibitor)

As a GSK-3β inhibitor, any GSK-3β inhibitor that inhibits GSK (glycogen synthase kinase)-3 can be used, and CHIR99021 is preferable. The concentration of the GSK-3β inhibitor (in the case of CHIR99021) added to the culture system is, for example, 0.1 to 20 µM, preferably 0.5 to 10 µM, and more preferably 1 to 5 µM. It is to be noted that when a compound different from CHIR99021 is used as a GSK-3β inhibitor, the concentration thereof added to the culture system can be set by those skilled in the art in accordance with to the above-described concentration range, taking into consideration the differences between the characteristics of the GSK-3β inhibitor used and those of CHIR99021 (especially, activity).Moreover, whether or not the set concentration range is appropriate can be confirmed by preliminary experiments.

### (Epidermal growth factor)

The Epidermal growth factor (EGF) is a protein characterized in that it binds to the epidermal growth factor receptor (EGFR) present on the cell surface of various cells, thereby inducing cell growth and proliferation. The epidermal growth factor is also sometimes referred to as an epidermal proliferation factor, an epidermal cell growth factor, or an epidermal cell proliferation factor, etc. In the present invention, EGF preferably has a human amino acid sequence, but is not particularly limited. EGF may be produced from human cells or animal cells by a genetic recombination technology, or it may be a peptide compound. The peptide compound may be a chemically synthesized peptide compound that has an activity equivalent to or greater than that of a peptide derived from a living organism. The peptide compound is sometimes referred to as an alternative peptide compound. Some peptide compounds will be further developed in the future, and their use by those skilled in the art is naturally anticipated.

The concentration of EGF added to the culture system may be, for example, 3 to 100 ng/mL, preferably 5 to 50 ng/mL, and more preferably 10 to 30 ng/mL. In the present invention, EGF preferably has a human amino acid sequence, but is not particularly limited.

### (Hepatocyte growth factor)

The hepatocyte growth factor (HGF) is a protein characterized in that it binds to the receptor tyrosine kinase Met, thereby inducing biological activities such as cell proliferation, survival promotion, and migration. In the present invention, HGF preferably has a human amino acid sequence, but is not particularly limited. HGF may be produced from human cells or animal cells by a genetic recombination technology, or it may be an alternative peptide compound. An example of the alternative peptide compound for HGF may be PG-001, which is available from PeptiGrowth.

The concentration of HGF added to the culture system may be, for example, 1 to 200 ng/mL, preferably 3 to 100 ng/mL, more preferably 10 to 50 ng/mL, and further preferably 15 to 30 ng/mL.

### (Sphingophospholipid)

The sphingophospholipid is a compound in which a phosphate group binds to a sphingoid base (a long-chain aliphatic amino acid having a hydroxyl group). Examples of the sphingophospholipid may include sphingosine-1-phosphate (S1P), sphingomyelin, and their salts. In a preferred aspect, the sphingophospholipid is S1P having the structure shown below, or a salt thereof.

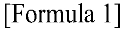

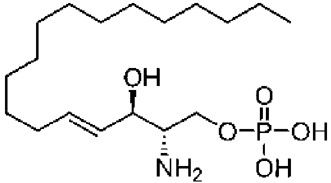

The concentration of the sphingophospholipid (in the case of S1P) added to the culture system is, for example, 0.01 to 50 µM, preferably 0.05 to 30 µM, more preferably 0.1 to 10 µM, and further preferably 0.5 to 5 µM. It is to be noted that when a sphingophospholipid different from S1P is used, the concentration thereof added to the culture system can be set by those skilled in the art in accordance with to the above-described concentration range, taking into consideration the differences between the characteristics of the sphingophospholipid used and those of S1P (especially, activity). Moreover, whether or not the set concentration range is appropriate can be confirmed by preliminary experiments.

### (Glycerophospholipid)

The glycerophospholipid is a compound in which phosphoric acid and fatty acid each bind to glycerol via hydroxyl groups. In the glycerophospholipid of the present invention, the hydroxyl groups, to which the phosphoric acid and the fatty acid each bind, may be at any position on the glycerol. Examples of the glycerophospholipid may include lysophosphatidic acid (LPA), phosphatidic acid, phosphatidylcholine, phosphatidylethanolamine, phosphatidylserine, phosphatidylinositol, phosphatidylglycerol, and salts thereof. In a preferred aspect, the glycerophospholipid is LPA having the structure shown below, or a salt thereof.

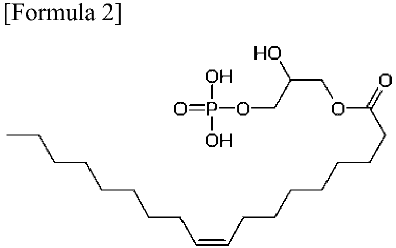

The concentration of the glycerophospholipid (in the case of LPA) added to the culture system is, for example, 0.05 to 100 µM, preferably 0.1 to 50 µM, more preferably 0.5 to 30 µM, and further preferably 1 to 10 µM. It is to be noted that when a glycerophospholipid different from LPA is used, the concentration thereof added to the culture system can be set by those skilled in the art in accordance with to the above-described concentration range, taking into consideration the differences between the characteristics of the glycerophospholipid used and those of LPA (especially, activity). Moreover, whether or not the set concentration range is appropriate can be confirmed by preliminary experiments.

When either one or both of the sphingophospholipid and the glycerophospholipid are salts, the salts can be acidic or basic salts. Examples of the basic salts may include: alkali metal salts such as sodium and potassium; alkaline earth metal salts such as calcium and magnesium; ammonium salts; and nitrogen-containing organic base salts such as trimethylamine, triethylamine, tributylamine, pyridine, N,N-dimethylaniline, N-methylpiperidine, N-methylmorpholine, diethylamine, dicyclohexylamine, procaine, dibenzylamine, N-benzyl-β-phenethylamine, 1-efenamine, and N,N'-dibenzylethylenediamine, but the examples are not limited thereto. Examples of the acidic salts may include: mineral salts such as hydrochloride, hydrobromide, nitric acid, and sulfuric acid; organic carboxylates such as formic acid, acetic acid, citric acid, oxalic acid, fumaric acid, maleic acid, succinic acid, malic acid, tartaric acid, aspartic acid, trichloroacetic acid, and trifluoroacetic acid; and sulfonates such as methanesulfonic acid, benzenesulfonic acid, p-toluenesulfonic acid, mesitylenesulfonic acid, and naphthalenesulfonic acid, but the examples are not limited thereto.

### (Other components)

The culture of (ii) may be a culture performed in the presence of any given appropriate components, in addition to each of the factors described above. Examples of such appropriate components may include, but are not limited to, serum, antibiotics, amino acids, vitamins, growth factors, and ROCK inhibitors.

The serum may be, for example, mammal-derived serum, and is preferably fetal bovine serum (FBS). The concentration of FBS added to the culture system may be, for example, 0.05% to 20%, and is preferably 0.1% to 10%, and more preferably 0.5% to 5%. In addition, as a serum replacement, KSR (KnockOut^{™} Serum Replacement, Gibco), or a B27 supplement, from which vitamin A is removed, etc., can be used. As a B27 supplement from which vitamin A is removed, for example, commercially available B-27^{™} Supplement (50X), minus vitamin A (Gibco) can be used.

The antibiotics may be, for example, penicillin, streptomycin, ampicillin, puromycin, gentamicin, etc., and are preferably penicillin and streptomycin. The concentration of penicillin and streptomycin added to the culture system is, for example, addition of an amount of 1/100 (final concentration x 1), in the case of a commercially available penicillin-streptomycin solution for culture (x 100). In addition, the antibiotic may also have an antifungal action. The concentration of the antibiotic added to the culture system may be the concentration commonly used in cell culture.

Preferred examples of the amino acid may include essential amino acids such as L-alanine, L-asparagine, L-aspartic acid, L-glutamic acid, L-glycine, L-proline or L-serine, as well as L-alanyl-L-glutamine. The concentration of the amino acid added to the culture system is, for example, addition of an amount of 1/50 or 1/100 (final concentration x 1), in the case of a commercially available essential amino acid solution (x 50) or L-glutamine solution (x 100). The concentration of the amino acid added to the culture system may be the concentration commonly used in cell culture.

Examples of the vitamin may include vitamin C (ascorbic acid), vitamin B1 (thiamine), vitamin B2 (riboflavin), B3 (niacin), B5 (pantothenic acid), B6 (pyridoxine), B7 (biotin), B12 (cobalamin), folic acid, vitamin A, and vitamin E. In a preferred aspect, the culture system contains ascorbic acid or a derivative thereof. The ascorbic acid derivative is, for example, a phosphorylated form of ascorbic acid or a metal salt thereof. The concentration of the ascorbic acid or a derivative thereof added to the culture system is, for example, 1 to 100 ng/mL, preferably 3 to 50 ng/mL, and more preferably 5 to 30 ng/mL. The concentration of vitamins other than the ascorbic acid or a derivative thereof added to the culture system may be the concentration commonly used in cell culture.

In one preferred aspect, in the culture of (ii), the at least 5 types selected from a ROCK inhibitor, a TGFβ receptor inhibitor, a GSK-3β inhibitor, EGF, HGF, a sphingophospholipid, and a glycerophospholipid comprise a ROCK inhibitor, EGF, HGF, a sphingophospholipid, and a glycerophospholipid, or comprise at least 6 types selected from a ROCK inhibitor, a TGFβ receptor inhibitor, a GSK-3β inhibitor, EGF, HGF, a sphingophospholipid, and a glycerophospholipid. From the viewpoint of quickly obtaining cell-cell adhesion morphology, the culture of (ii) is preferably a culture performed in the presence of a ROCK inhibitor, a TGFβ receptor inhibitor, a GSK-3β inhibitor, EGF, HGF, a sphingophospholipid, and a glycerophospholipid.

In one aspect, the culture system in the culture of (ii) does not have a composition comprising any given combination of one or more types selected from the group consisting of nicotinamide, ascorbic acid-2 phosphate, dexamethasone, and 10% or more of FBS.

### (Culture period)

The period of the culture of (ii) is not particularly limited, and the lower limit thereof may be, for example, 3 days or more, 4 days or more, 5 days or more, preferably 6 days or more, more preferably 7 days or more, or 8 days or more, 9 days or more, 10 days or more, or even longer. On the other hand, the upper limit thereof may be, for example, 35 days or less, 30 days or less, 25 days or less, 20 days or less, 15 days or less, or 10 days or less, and is not particularly limited, as long as plateable hepatocytes can be kept alive. The period (range) may be expressed by combining any of the upper limits and any of the lower limits of the above-described periods.

### (i) Culture in the presence of at least 4 types selected from a ROCK inhibitor, EGF, HGF, a sphingophospholipid, and a glycerophospholipid

In one aspect, the method for producing plateable hepatocytes of the present invention comprises (i) culturing suspension hepatocytes in the presence of at least 4 types selected from a ROCK inhibitor, EGF, HGF, a sphingophospholipid, and a glycerophospholipid, before the culture of (ii). (i) "in the presence of at least 4 types selected from a ROCK inhibitor, EGF, HGF, a sphingophospholipid, and a glycerophospholipid" is synonymous with a culture system comprising at least 4 types selected from a ROCK inhibitor, EGF, HGF, a sphingophospholipid, and a glycerophospholipid (hereinafter, in the present invention, when the expression "in the presence of (predetermined components)" is used, it similarly means a culture system comprising (predetermined components)). In the present invention, the culture system may include a medium, a culture medium, a culture vessel, etc. In one aspect, the expression "to culture in the presence of at least 4 types selected from a ROCK inhibitor, EGF, HGF, a sphingophospholipid, and a glycerophospholipid" means to culture in a medium comprising at least 4 types selected from a ROCK inhibitor, EGF, HGF, a sphingophospholipid, and a glycerophospholipid. The medium is not particularly limited, as long as it can be generally used in the culture of hepatocytes. The medium may be, for example, Dulbecco's Modified Eagle Medium (DMEM).

Each of the ROCK inhibitor, EGF, HGF, sphingophospholipid, and glycerophospholipid, or any other components, in the culture of (i), can be selected as described for the culture of (ii), but the conditions do not need to be the same as those for the culture of (ii).

The culture of (i) is preferably a culture performed in the presence of a ROCK inhibitor, EGF, HGF, a sphingophospholipid, and a glycerophospholipid, from the viewpoint of quickly obtaining cell-cell adhesion morphology.

In one aspect, the culture system in the culture of (i) does not have a composition comprising any given combination of one or more types selected from the group consisting of Y27632, A83-01, CHIR99021, nicotinamide, ascorbic acid-2 phosphate, dexamethasone, and 10% or more of FBS.

### (Culture period)

The period of the culture of (i) is not particularly limited, and the lower limit thereof may be, for example, 30 minutes or more, 1 hour or more, preferably 2 hours or more, more preferably 3 hours or more, or 5 hours or more, 10 hours or more, 15 hours or more, 20 hours or more, and further preferably 24 hours or more. On the other hand, the upper limit thereof may be, for example, 5 days or less, 4 days or less, 3 days or less, preferably 2 days or less, more preferably 1 day (24 hours) or less, or 20 hours or less, 15 hours or less or 10 hours or less. The period (range) may be expressed by combining any of the upper limits and any of the lower limits of the above-described periods.

### Method comprising formation of spheroids

In one aspect, the culture of (ii) comprises forming spheroids from suspension hepatocytes. By allowing the culture to comprise the formation of spheroids, it becomes possible to reduce the culture period and to reduce the culture of (i).

Spheroids can be formed by appropriate methods known to those skilled in the art, and are generally formed by a three-dimensional culture using a culture vessel equipped with a low-cell-adhesion or non-cell-adhesion culture surface (for example, a culture surface to which low-cell-adhesion or non-cell-adhesion properties have been imparted by treatment/binding of polymer materials or hydrogels). Such a culture vessel may be a culture vessel in which multiple wells of uniform shape and size are formed on the culture surface (generally called pattern plates; specific examples may include EZSPHERE^{®} provided by AGC Techno Glass Co., Ltd. and Elplasia provided by Kuraray Co., Ltd.).

The period of the culture of (ii) can be 6 days or less, 5 days or less, 4 days or less, or 3 days or less, if the culture of (ii) comprises the formation of spheroids, and may be even shorter than that described above, as long as it is possible to form spheroids.

The culture of (ii) may further comprise conversion of spheroids into single cells. Such conversion into single cells can be carried out, for example, using a cell dissociation solution. The cell dissociation solution may contain, for example, proteolytic enzymes such as trypsin-EDTA, collagenase IV, or metalloproteinase, either alone or in appropriate combinations, and is preferably one having low cytotoxicity. Such a cell dissociation solution may be TrypLE (Invitrogen) or Accutase (Millipore).

### [Passage]

After the culture of (ii), a passage operation may be performed. Conventionally, it has been generally difficult to perform a subculture in the culture of plateable hepatocytes from human frozen hepatocytes. Accordingly, the ability to subculture the plateable hepatocytes obtained in the present invention is an advantage. The number of passage operations during the culture period is, for example, 1 to 5 times, preferably 1 to 3 times, more preferably 1 or 2 times. The passage operation may be performed according to methods known to those skilled in the art, for example, when the cells become confluent or subconfluent, a portion of the cells is collected and transferred to another culture vessel, and the culture is continued. A cell dissociation solution, etc. may be utilized for the recovery of the cells. As such a cell dissociation solution, for example, proteolytic enzymes such as trypsin-EDTA, collagenase IV, or metalloproteinase can be used either alone or in appropriate combinations. A cell dissociation solution having low cytotoxicity is preferable. Such a cell dissociation solution, for example, a commercially available product such as Dispase (EIDIA Co., Ltd.), TrypLE (Invitrogen), or Accutase (Millipore) can be obtained.

### [Cryopreservation]

In one aspect, the method for producing plateable hepatocytes of the present invention may comprise cryopreserving hepatocytes and further thawing them, after the culture of (ii). Conventionally, it has been generally difficult to cryopreserve plateable hepatocytes and then to thaw them for use. Accordingly, the plateable hepatocytes obtained in the present invention is advantageous in that the present plateable hepatocytes can be cultured after cryopreserving and thawing them. Cryopreservation may be performed according to methods known to those skilled in the art, for example, by suspending the cells to an appropriate cell density using a medium suitable for cryopreservation, such as a complete medium containing a cryoprotective reagent such as dimethyl sulfoxide (DMSO), placing the suspension in a vessel for cryopreservation, such as a cryovial, and then storing it at -80°C or below. The cryopreservation period is not particularly limited, as long as the function of the plateable hepatocytes is not impaired. Thawing can be performed according to methods known to those skilled in the art, for example, by transferring the cryopreservation vessel containing the cells to a 37°C water bath and gently swirling it.

### (iii) Culture in the presence of at least 9 types selected from a ROCK inhibitor, a TGFβ receptor inhibitor, a GSK-3β inhibitor, a cAMP signaling activator, EGF, HGF, a sphingophospholipid, a glycerophospholipid, a γ secretase inhibitor, oncostatin M, and dexamethasone

In one aspect, the method for producing plateable hepatocytes of the present invention comprises: (ii) culturing suspension hepatocytes in the presence of at least 5 types selected from a ROCK inhibitor, a TGFβ receptor inhibitor, a GSK-3β inhibitor, EGF, HGF, a sphingophospholipid, and a glycerophospholipid; and then, (iii) culturing the resulting hepatocytes in the presence of at least 9 types selected from a ROCK inhibitor, a TGFβ receptor inhibitor, a GSK-3β inhibitor, a cAMP signaling activator, EGF, HGF, a sphingophospholipid, a glycerophospholipid, a γ secretase inhibitor, oncostatin M, and dexamethasone. It is considered that the culture of (iii) is not essential for obtaining plateable hepatocytes from suspension hepatocytes, but that the culture of (iii) can mature the plateable hepatocytes obtained in the culture of (ii).

Individual factors, i.e., the ROCK inhibitor, TGFβ receptor inhibitor, GSK-3β inhibitor, EGF, HGF, sphingophospholipid, and glycerophospholipid, or any other components, in the culture of (iii), can be selected or determined in the same manners as those as described for the cultures of (i) and (ii). It is to be noted that such selection or determination may be carried out independently from the cultures of (i) and (ii).

The TGFβ receptor inhibitor can be selected or determined, independently from the cultures of (i) and (ii) described above, in the same manners as those for the cultures of (i) and (ii). The TGFβ receptor inhibitor is particularly preferably one or more types selected from A83-01 and SB431542. In addition, an alternative peptide compound having a TGFβ inhibitory action may be used instead of the TGFβ receptor inhibitor. The concentration of the TGFβ receptor inhibitor added to the culture system may be, for example, 0.05 to 10 µM, preferably 0.1 to 5 µM, and more preferably 0.5 to 3 µM, in the case of A83-01. Moreover, in the case of SB431542, the concentration thereof added to the culture system may be, for example, 0.5 to 50 µM, preferably 1 to 30 µM, and more preferably 5 to 20 µM. When a compound different from A83-01 or SB431542 is used, the additive concentration can be set by those skilled in the art in accordance with the above-described concentration range, taking into consideration the differences between the characteristics of the compound used and those the exemplified compounds (especially, differences in activity). Moreover, whether or not the set concentration range is appropriate can be confirmed by preliminary experiments in accordance with the after-mentioned Examples.

Any γ secretase inhibitor can be used, as long as it has an action to inhibit γ secretase. Examples of the γ secretase inhibitor may include DAPT (N-[N-(3,5-difluorophenacetyl-L-alanyl)]-(S)-phenylglycine t-butyl ester) and dibenzazepine, with DAPT being preferable. The concentration of the γ secretase inhibitor (in the case of DAPT) added to the culture system may be, for example, 0.1 to 50 µM, preferably 0.5 to 30 µM, and more preferably 0.1 to 20 µM. It is to be noted that when a γ secretase inhibitor other than DAPT is used, the concentration thereof added to the culture system can be set by those skilled in the art in accordance with the above-described concentration range, taking into consideration the differences between the characteristics of the γ secretase inhibitor used and those of DAPT (especially, activity). Moreover, whether or not the set concentration range is appropriate can be confirmed by preliminary experiments.

The concentration of oncostatin M added to the culture system may be, for example, 1 to 50 ng/mL, preferably 5 to 40 ng/mL, and more preferably 10 to 30 ng/mL. In addition, the concentration of dexamethasone added to the culture system may be, for example, 0.1 to 50 µM, preferably 0.5 to 30 µM, and more preferably 0.1 to 20 µM.

The cAMP signaling activator may be one or more selected from the group consisting of a cAMP derivative, a cAMP-degrading enzyme inhibitor, and a cAMP activator, with a cAMP activator being preferable.

Examples of the cAMP derivative that can be used herein may include PKA activators (e.g., 8-Br-cAMP (8-Bromoadenosine-3',5'-cyclic monophosphate sodium salt, CAS Number: 76939-46-3), 6-Bnz-cAMP (N6-Benzoyladenosine-3',5'-cyclic monophosphate sodium salt, CAS Number: 1135306-29-4), cAMPS-Rp ((R)-Adenosine, cyclic 3',5'-(hydrogenphosphorothioate)triethylammonium salt, CAS Number: 151837-09-1), cAMPS-Sp ((S)-Adenosine, cyclic 3',5'-(hydrogenphosphorothioate)triethylammonium salt, CAS Number: 93602-66-5), Dibutyryl-cAMP (N6,O2'-Dibutyryl adenosine 3',5'-cyclic monophosphate sodium salt, CAS Number: 16980-89-5), and 8-Cl-cAMP (8-Chloroadenosine-3',5'-cyclic monophosphate salt, CAS Number: 124705-03-9)), and Epac activators (Rp-8-Br-cAMPS (8-Bromoadenosine 3',5'-cyclic Monophosphothioate, Rp-Isomer. sodium salt, CAS Number: 129735-00-8), 8-CPT-cAMP (8-(4-Chlorophenylthio)adenosine 3',5'-cyclic monophosphate, CAS Number: 93882-12-3), 8-pCPT-2'-O-Me-cAMP (8-(4-Chlorophenylthio)-2'-O-methyladenosine 3',5'-cyclic monophosphate monosodium, CAS Number: 634207-53-7), etc.). The concentration of the cAMP derivative added (in the case of 8-Br-cAMP) may be, for example, 0.1 mM to 10 mM, preferably 0.2 mM to 5 mM, and more preferably 0.5 mM to 2 mM. It is to be noted that when a compound different from the exemplified compound, namely, 8-Br-cAMP, is used, the additive concentration thereof can be set by those skilled in the art in accordance with the above-described concentration range, taking into consideration the differences between the characteristics of the compound used and those of the exemplified compound (8-Br-cAMP) (especially, differences in activity). Moreover, whether or not the set concentration range is appropriate can be confirmed by preliminary experiments.

Examples of the cAMP-degrading enzyme inhibitor may include IBMX (3-isobutyl-1-methylxanthine) (MIX), Theophylline, Papaverine, Pentoxifylline (Trental), KS-505, 8-Methoxymethyl-IBMX, Vinpocetine (TCV-3B), EHNA, Trequinsin (HL-725), Lixazinone (RS-82856), (LY-186126), Cilostamide (OPC3689), Bemoradan (RWJ-22867), Anergrelide (BL4162A), Indolidan (LY195115), Cilostazol (OPC-13013), Milrinone (WIN47203), Siguazodan (SKF-94836), 5-Methyl-imazodan (CI 930), SKF-95654, Pirilobendan (UD-CG 115 BS), Enoximone (MDL 17043), Imazodan (CL 914), SKF-94120, Vesnarinone (OPC 8212), Rolipram (Ro-20-1724), (ZK-62711), Denbufyll'ine, Zaprinast (M&B-22, 948), Dipyridamole, Zaprinast (M&B-22, 948), Dipyridamole, Zardaverine, AH-21-132, and Sulmazol (AR-L 115 BS). The concentration of the cAMP-degrading enzyme inhibitor added (in the case of IBMX) may be, for example, 0.05 mM to 5 mM, preferably 0.1 mM to 3 mM, and more preferably 0.2 mM to 1 mM. It is to be noted that when a compound different from the exemplified compound, namely, IBMX, is used, the additive concentration thereof can be set by those skilled in the art in accordance with the above-described concentration range, taking into consideration the differences between the characteristics of the compound used and those of the exemplified compound (IBMX) (especially, differences in activity). Moreover, whether or not the set concentration range is appropriate can be confirmed by preliminary experiments.

Examples of the cAMP activator may include forskolin, indomethacin, NKH477 (colforsin daropate), cell-derived toxin proteins (pertussis toxin and cholera toxin), PACAP-27, PACAP-38, and SKF83822, with forskolin being preferable. The concentration of the cAMP activator added to the culture system (in the case of forskolin) may be, for example, 1 to 50 µM, preferably 3 to 30 µM, and more preferably 5 to 15 µM. It is to be noted that when a compound different from the exemplified compound, namely, forskolin, is used, the additive concentration thereof can be set by those skilled in the art in accordance with the above-described concentration range, taking into consideration the differences between the characteristics of the compound used and those of the exemplified compound (forskolin) (especially, differences in activity). Moreover, whether or not the set concentration range is appropriate can be confirmed by preliminary experiments in accordance with the after-mentioned Examples.

In one preferred aspect, in the culture of (iii), the at least 9 types selected from a ROCK inhibitor, a TGFβ receptor inhibitor, a GSK-3β inhibitor, a cAMP signaling activator, EGF, HGF, a sphingophospholipid, a glycerophospholipid, a γ secretase inhibitor, oncostatin M, and dexamethasone comprise a ROCK inhibitor, a cAMP signaling activator, EGF, HGF, a sphingophospholipid, a glycerophospholipid, a γ secretase inhibitor, oncostatin M, and dexamethasone, or comprise at least 10 types selected from a ROCK inhibitor, a TGFβ receptor inhibitor, a GSK-3β inhibitor, a cAMP signaling activator, EGF, HGF, a sphingophospholipid, a glycerophospholipid, a γ secretase inhibitor, oncostatin M, and dexamethasone. From the viewpoint of quickly obtaining cell-cell adhesion morphology, the culture of (iii) is preferably a culture performed in the presence of a ROCK inhibitor, a TGFβ receptor inhibitor, a GSK-3β inhibitor, a cAMP signaling activator, EGF, HGF, a sphingophospholipid, a glycerophospholipid, a γ secretase inhibitor, oncostatin M, and dexamethasone.

In one aspect, the culture system in the culture of (iii) does not have a composition comprising any given combination of one or more types selected from the group consisting of nicotinamide and ascorbic acid 2-phosphate.

### (Culture period)

The period of the culture of (iii) is not particularly limited, and the lower limit thereof may be, for example, 12 hours or more, 1 day or more, and preferably 2 days or more. On the other hand, the upper limit thereof may be, for example, 10 days or less, 9 days or less, 8 days or less, 7 days or less, 6 days or less, 5 days or less, 4 days or less, or 3 days or less. The period (range) may be expressed by combining any of the upper limits and any of the lower limits of the above-described periods.

### (Culture surface)

Each of the cultures of (i) to (iii) can be carried out on a culture surface suitable for the present invention. The culture surface is not particularly limited, as long as it can be generally used for cell culture. Such a culture surface may be, for example, a cell culture plate, flask, petri dish, dish, and cell culture insert. Moreover, the culture surface may also be coated with any component suitable for cell differentiation, proliferation, adhesion, etc. In a preferred aspect, the culture can be performed on a culture surface coated with a basement membrane component. Examples of the basement membrane components may include laminin, type IV collagen, entactin, heparan sulfate proteoglycan, vitronectin, fibronectin, and fragments thereof. Examples of products containing these basement membrane components may include Matrigel (Corning) and iMatrix-511 (Matrixome, Inc.), which contains the human laminin 511E8 fragment. Furthermore, the cultures involving the culture of (i) and the culture of (ii) can also be performed on culture devices commonly known as Organ-on-a-chip (Microphysiological System; MPS), etc.

### (Others)

Other culture conditions can be those commonly used in the culture of animal cells, and may be, for example, in the environment of 37°C and 5% CO₂. The basal medium may be any medium generally suitable for the culture of animal cells, and examples of the basal medium that can be used herein may include Iskov-modified Dulbecco's medium (IMDM) (Gibco, etc.), Hamm F12 medium (SIGMA, Gibco, etc.), Dulbecco's Modified Eagle Medium (DMEM) (Nacalai Tesque, Sigma, Gibco, etc.), Glasgow basal medium (Gibco, etc.), and RPMI1640 medium. A preferred example of the basal medium may be DMEM/F12 (a mixed medium of DMEM and Hamm F12 medium).

### < Method for evaluating metabolism of test substance >

In one aspect, the present invention is a method for evaluating the metabolism of a test substance, comprising: (1) a step of obtaining plateable hepatocytes by the method for producing plateable hepatocytes of the present invention; (2) a step of allowing a test substance to come into contact with the plateable hepatocytes obtained in the step (1); and (3) a step of measuring the metabolism of the test substance.

The test substance may be either an organic compound or an inorganic compound of various molecular sizes. Examples of the organic compound may include nucleic acids, peptides, proteins, lipids (simple lipids, complex lipids (phosphoglyceride, sphingolipid, glycosylglyceride, cerebroside, etc.), prostaglandin, isoprenoid, terpene, steroid, polyphenol, catechin, and vitamins (B1, B2, B3, B5, B6, B7, B9, B12, C, A, D, E, etc.)). Existing components or candidate components of medicaments, nutritional foods, etc. are also preferred test substances. Otherwise, plant extracts, cell extracts, culture supernatants, etc., may also be used as test substances. By adding two or more types of test substances simultaneously, interactions, synergistic effects, etc. between the test substances may be investigated. The test substance may be derived from natural products, or may also be synthesized. In the latter case, for example, an efficient assay system can be constructed utilizing combinatorial synthesis methods.

The period in which the test substance is allowed to come into contact with plateable hepatocytes in the step (2) can be arbitrarily set by those skilled in the art, depending on the type and amount of the test substance, measurement conditions, etc. The contact period may be, for example, 10 minutes to 3 days, 1 hour to 1 day, etc. The contact may also be performed dividedly over multiple times.

The measurement of the metabolism of the test substance in the step (3) can be performed by any method known to those skilled in the art, and it may be, for example, a method for measuring the amount of the metabolite of the test substance after the step (2). The metabolite of the test substance is a substance produced when the test substance is metabolized in the liver, and is known to those skilled in the art. As an example, when Midazolam is used as such a test substance as shown in the Examples, its hepatic metabolite may be 1-hydroxymidazolam. As methods of measuring the amount of the metabolites, any methods may be selected by those skilled in the art, and examples of such methods may include mass spectrometry, liquid chromatography, liquid chromatography-mass spectrometry (LC-MS, LC-MS/MS), and immunological methods (for example, fluorescence immunoassay (FIA) and enzyme immunoassay (EIA)). If large amounts of metabolites are produced, it can be evaluated that the test substance has been metabolized more.

### < Method for inducing metabolic enzyme to hepatocytes >

In one aspect, the present invention is a method for inducing a metabolic enzyme to hepatocytes, comprising: (1) a step of obtaining plateable hepatocytes by the method for producing plateable hepatocytes of the present invention; and (2) a step of allowing a metabolic enzyme inducer to come into contact with the plateable hepatocytes obtained in the step (1). Since it is difficult to use suspension hepatocytes in metabolic enzyme induction assays, the metabolic enzyme induction assays can be efficiently carried out by obtaining plateable hepatocytes by the present invention.

Metabolic enzymes in hepatocytes are, for example, drug metabolic enzymes, and examples thereof may include cytochrome P450 (CYP) family, uridine diphosphate-glucuronosyltransferase (UGT), and sulfotransferase (SULT). In particular, the CYP family is a main drug metabolic enzyme in hepatocytes. The CYP family is classified based on amino acid sequence homology, and many drugs are known as substrates for CYP1A2, CYP2B6, CYP2C9, CYP2C19, CYP3A4, etc. Moreover, drugs that induce the expression of hepatocyte metabolic enzymes in hepatocytes (metabolic enzyme inducers) are known to those skilled in the art. For example, omeprazole, phenobarbital, rifampicin, etc. are known as metabolic enzyme inducers for CYP1A2, CYP2B6, and CYP3A4, respectively.

The time in which the metabolic enzyme inducer is allowed to come into contact with plateable hepatocytes in the step (2) can be appropriately set by those skilled in the art, depending on the types and amounts of the metabolic enzyme and the metabolic enzyme inducer, measurement conditions, etc. The contact may also be performed dividedly over multiple times.

The fact that a metabolic enzyme were induced to hepatocytes can be confirmed by methods known to those skilled in the art. For example, the gene expression of a metabolic enzyme may be measured, both when a metabolic enzyme inducer has been allowed to come into contact with plateable hepatocytes and when it has not been allowed to come into contact therewith, and a comparison may be then made. Alternatively, the activity of the metabolic enzyme may be measured in both cases and a comparison may be then made. The measurement of gene expression can be performed by methods known to those skilled in the art, such as, for example, quantitative PCR. The measurement of the activity of a metabolic enzyme can be performed, for example, by using a substrate specific to a target metabolic enzyme, and allowing the cells to react with the metabolic enzyme under predetermined conditions, and measuring the metabolite.

In one aspect, the method for inducing a metabolic enzyme to hepatocytes of the present invention comprises subjecting the plateable hepatocytes obtained in the step (1) to a sandwich culture. The sandwich culture is a method of culturing cells by sandwiching them with extracellular matrix components. It is known that hepatocyte-specific functions such as drug-metabolizing activity are improved by the sandwich culture. Moreover, it is known that a three-dimensional structure is formed by the sandwich culture, so that hepatocytes can be maintained for a long period of time. Furthermore, it is known that efflux transporters are located on the bile duct lateral membrane by the sandwich culture, so that biliary excretion of drugs can be evaluated. Non-restrictive examples of extracellular matrix components that can be used in the sandwich culture may include collagen gel and Matrigel.

### < Method for producing hepatocyte spheroids >

In one aspect, the present invention is a method for producing hepatocyte spheroids, comprising: (1) a step of obtaining plateable hepatocytes by the method for producing plateable hepatocytes of the present invention; and (2) a step of culturing the plateable hepatocytes obtained in the step (1) to form hepatocyte spheroids. In general, cell-to-cell adhesion has been difficult, once plateable hepatocytes have been detached from the culture surface. However, since the plateable hepatocytes obtained by the present invention can maintain such cell-to-cell adhesion even after being detached from the culture surface, the present plateable hepatocytes are advantageous in that hepatocyte spheroids can be produced by a three-dimensional culture.

In the step (2) of culturing the plateable hepatocytes to form hepatocyte spheroids, a suspension culture is suitable. In the suspension culture, in general, a culture vessel equipped with a low-cell-adhesion or non-cell-adhesion culture surface (for example, a culture surface to which low-cell-adhesion or non-cell-adhesion properties have been imparted by treatment/binding of polymer materials or hydrogels) is used, and cells are cultured in a state away from the culture surface (i.e., in a suspended state). The culture vessel used for the suspension culture is not particularly limited, and examples of the culture vessel that can be used herein may include a dish, a flask, a multi-well plate, a tube, a tray, and a culture bag. A culture vessel with multiple wells of uniform shape and size formed on the culture surface (generally called a pattern plate; specific examples may include EZSPHERE^{®} provided by AGC Techno Glass Co., Ltd. and Elplasia provided by Kuraray Co., Ltd.) is used to form multiple spheroids together. In this way, spheroids can be formed efficiently. It is to be noted that the composition of the culture medium used in the step (2) is not particularly limited as long as it is suitable for forming hepatocyte spheroids, but that the medium may be the same medium as that used in the step (1) for producing plateable hepatocytes.

In one aspect, the culture performed in the step (2) is a sandwich culture.

The period of the step (2) (culture period) may be, for example, 10 to 35 days, preferably 12 to 30 days, and more preferably 15 to 25 days. If this period is too short, spheroids of sufficient size will not be formed. On the other hand, if this period is too long, the spheroids may grow larger than necessary, and the cells inside may cause necrosis.

### < Method for subjecting hepatocyte spheroids to two-dimensional culture >

In one aspect, the present invention is a method for converting hepatocyte spheroids into single cells, and dissociate hepatocytes into single cells to a two-dimensional culture, wherein the method comprises: (1) a step of obtaining plateable hepatocytes by the method for producing plateable hepatocytes of the present invention; (2) Dissociating the plateable hepatocytes obtained in the step (1) into single cells; and (3) a step of culturing the hepatocytes dissociated into single cells in the step (2), so as to produce hepatocyte spheroids. In general, once plateable hepatocytes have been dissociated into single cells (i.e. changed to a state in which cells do not adhere to one another), it has been difficult to allow the cells to adhere to one another again). On the other hand, since the plateable hepatocytes obtained by the present invention can adhere to one another even after being converted into single cells, the present plateable hepatocytes are advantageous in that hepatocyte spheroids can be produced from them, the produced hepatocyte spheroids can be converted into single cells, which can be then subjected to a two-dimensional development culture.

The dissociation of plateable hepatocytes into single cells in the step (2) can be carried out, for example, using a cell dissociation solution. The cell dissociation solution may contain, for example, proteolytic enzymes such as trypsin-EDTA, collagenase IV, or metalloproteinase, either alone or in appropriate combinations, and is preferably one having low cytotoxicity. Such a cell dissociation solution may be TrypLE (Invitrogen) or Accutase (Millipore).

In one aspect, the dissociation into single cells in the step (2) may also be carried out using the hepatocyte spheroids obtained by the present invention.

In one aspect, the culture of the step (2) is a sandwich culture.

The step (3) of subjecting the hepatocyte spheroids converted into single cells to a two-dimensional culture, which comprises a step of culturing the hepatocytes that have been converted into single cells in the step (2), can be carried out on any given culture surface. The culture surface is not particularly limited as long as it can generally be used for cell culture, and examples of the culture surface may include a cell culture plate, flask, petri dish, dish, and cell culture insert (e.g., Transwell). A culture surface coated with a basement membrane component is preferable. Examples of the basement membrane components may include laminin, type IV collagen, entactin, heparan sulfate proteoglycan, vitronectin, fibronectin, and fragments thereof. An example of products containing these basement membrane components may be Matrigel (Corning).

The composition of the medium used in the step (3) is not particularly limited, as long as it is suitable for dissociating hepatocyte spheroids into single cells followed by subjecting them to a two-dimensional culture, which comprise a step of producing the hepatocyte spheroids. The medium used in the step (3) may be the same as that used in the step (1) of producing plateable hepatocytes.

### < Medium >

In one aspect, the present invention relates to a medium for use in the method for producing plateable hepatocytes of the present invention. In one aspect, the medium of the present invention comprises at least 4 types selected from a ROCK inhibitor, EGF, HGF, a sphingophospholipid, and a glycerophospholipid (hereinafter, this medium is referred to as medium (i)). In another aspect, the medium of the present invention comprises at least 5 types selected from a ROCK inhibitor, a TGFβ receptor inhibitor, a GSK-3β inhibitor, EGF, HGF, a sphingophospholipid, and a glycerophospholipid (hereinafter, this medium is referred to as medium (ii)).

The concentration of HGF in the medium of the present invention may be, for example, 1 to 200 ng/mL, preferably 3 to 100 ng/mL, more preferably 10 to 50 ng/mL, and further preferably 15 to 30 ng/mL. (Hereinafter, when the term "the medium of the present invention" is used, unless otherwise specified, it refers to one or more of the medium (i) and the medium (ii). It is to be noted that even when the term "the medium of the present invention" is used, it does not stipulate that the medium (i) and the medium (ii) must be under the same conditions, and the composition of the medium (i) and the composition of the medium (ii) are determined independently from each other.).

EGF in the medium of the present invention may be selected, as described in the explanation for the method for producing plateable hepatocytes in the present specification. The concentration of EGF in the medium of the present invention may be, for example, 3 to 100 ng/mL, preferably 5 to 50 ng/mL, and more preferably 10 to 30 ng/mL, in the medium.

The sphingophospholipid in the medium of the present invention may be selected, as described in the explanation for the method for producing plateable hepatocytes in the present specification. The concentration of the sphingophospholipid (in the case of S1P) in the medium of the present invention may be, for example, 0.01 to 50 µM, preferably 0.05 to 30 µM, more preferably 0.1 to 10 µM, and further preferably 0.5 to 5 µM, in the medium. It is to be noted that when a sphingophospholipid different from S1P is used, the concentration thereof in the medium can be set by those skilled in the art in accordance with the above-described concentration range, taking into consideration the differences between the characteristics of the sphingophospholipid used and those of the S1P (especially, activity). Moreover, whether or not the set concentration range is appropriate can be confirmed by preliminary experiments.

The glycerophospholipid in the medium of the present invention may be selected, as described in the explanation for the method for producing plateable hepatocytes in the present specification. The concentration of the glycerophospholipid (in the case of LPA) in the medium of the present invention may be, for example, 0.05 to 100 µM, preferably 0.1 to 50 µM, more preferably 0.5 to 30 µM, and further preferably 1 to 10 µM, in the medium. It is to be noted that when a glycerophospholipid different from LPA is used, the concentration thereof in the medium can be set by those skilled in the art in accordance with the above-described concentration range, taking into consideration the differences between the characteristics of the glycerophospholipid used and those of the LPA (especially, activity). Moreover, whether or not the set concentration range is appropriate can be confirmed by preliminary experiments.

The ROCK inhibitor in the media (i) and (ii) may be selected, as described in the explanation for the method for producing plateable hepatocytes in the present specification. The concentration of the ROCK inhibitor (in the case of Y27632) in the medium of the present invention may be, for example, 0.1 to 50 µM, preferably 0.5 to 30 µM, and more preferably 1 to 20 µM, in the medium. It is to be noted that when a ROCK inhibitor different from Y27632 is used, the concentration thereof in the medium can be set by those skilled in the art in accordance with the above-described concentration range, taking into consideration the differences between the characteristics of the ROCK inhibitor used and those of the Y27632 (especially, activity). Moreover, whether or not the set concentration range is appropriate can be confirmed by preliminary experiments.

The TGFβ receptor inhibitor in the medium (ii) may be selected, as described in the explanation for the method for producing plateable hepatocytes in the present specification. The concentration of the TGFβ receptor inhibitor (in the case of A83-01) in the medium of the present invention may be, for example, 0.05 to 10 µM, preferably 0.1 to 5 µM, and more preferably 0.5 µM to 3 µM, in the medium. When a compound different from A83-01 is used, the concentration thereof in the medium can be set by those skilled in the art in accordance with the above-described concentration range, taking into consideration the differences between the characteristics of the compound used and those of the A83-01 (especially, differences in activity). Moreover, whether or not the set concentration range is appropriate can be confirmed by preliminary experiments in accordance with the after-mentioned Examples.

The GSK-3β inhibitor in the medium (ii) may be selected, as described in the explanation for the method for producing plateable hepatocytes in the present specification. The concentration of the GSK-3β inhibitor (in the case of CHIR99021) in the medium of the present invention may be, for example, 0.1 to 20 µM, preferably 0.5 to 10 µM, and more preferably 1 to 5 µM, in the medium. It is to be noted that when a GSK-3β inhibitor different from CHIR99021 is used, the concentration thereof in the medium can be set by those skilled in the art in accordance with the above-described concentration range, taking into consideration the differences between the characteristics of the GSK-3β inhibitor used and those of the CHIR99021 (especially, activity). Moreover, whether or not the set concentration range is appropriate can be confirmed by preliminary experiments.

The medium of the present invention may further comprise components suitable for the method for producing plateable hepatocytes of the present invention. Examples of those components may include serum or a serum replacement, an antibiotic, an amino acid, a vitamin, a growth factor, a ROCK inhibitor, but are not limited thereto.

The serum or the serum replacement may be selected, as described in the explanation for the method for producing plateable hepatocytes in the present specification. The concentration of the serum (in the case of FBS) in the medium of the present invention may be, for example, 0.05% to 20%, preferably 0.1% to 10%, and more preferably 0.5% to 5%, in the medium.

The antibiotic may be selected, as described in the explanation for the method for producing plateable hepatocytes in the present specification. The concentration of the antibiotic (in the case of penicillin and streptomycin) in the medium of the present invention is addition of an amount of 1/100 (final concentration x 1), for example, in the case of a commercially available penicillin-streptomycin solution for culture (x 100). The concentration of the antibiotic added to the culture system may be the concentration commonly used in cell culture.

The amino acid may be selected, as described in the explanation for the method for producing plateable hepatocytes in the present specification. The concentration of the amino acid in the medium of the present invention is addition of an amount of 1/50 or 1/100 (final concentration x 1), for example, in the case of a commercially available essential amino acid solution (x 50) or L-glutamic acid solution (x 100). The concentration of the amino acid added to the culture system may be the concentration commonly used in cell culture.

The vitamin may be selected, as described in the explanation for the method for producing plateable hepatocytes in the present specification. The concentration of the vitamin (in the case of ascorbic acid or a derivative thereof) in the medium of the present invention may be, for example, 1 to 100 ng/mL, preferably 3 to 50 ng/mL, and more preferably 5 to 30 ng/mL, in the medium. The concentration of a vitamin other than the ascorbic acid or a derivative thereof in the medium may be the concentration that is common in those skilled in the art as a concentration for cell culture.

The ROCK inhibitor may be selected, as described in the explanation for the method for producing plateable hepatocytes in the present specification. The concentration of the ROCK inhibitor (in the case of Y27632) in the medium of the present invention may be, for example, 0.1 to 50 µM, preferably 0.5 to 30 µM, and more preferably 1 to 20 µM, in the medium. It is to be noted that when a ROCK inhibitor different from Y27632 is used, the concentration thereof in the medium can be set by those skilled in the art in accordance with the above-described concentration range, taking into consideration the differences between the characteristics of the ROCK inhibitor used and those of the Y27632 (especially, activity). Moreover, whether or not the set concentration range is appropriate can be confirmed by preliminary experiments.

From the viewpoint of quickly obtaining cell-cell adhesion morphology, the medium (i) preferably comprises a ROCK inhibitor, EGF, HGF, a sphingophospholipid, and a glycerophospholipid.

In one aspect, the medium (i) does not have a composition comprising any given combination of one or more types selected from the group consisting of Y27632, A83-01, CHIR99021, nicotinamide, ascorbic acid 2-phosphate, dexamethasone, and 10% or more of FBS.

In one preferred aspect, the medium (ii) comprises a ROCK inhibitor, EGF, HGF, a sphingophospholipid, and a glycerophospholipid, or comprises at least 6 types selected from a ROCK inhibitor, a TGFβ receptor inhibitor, a GSK-3β inhibitor, EGF, HGF, a sphingophospholipid, and a glycerophospholipid. From the viewpoint of quickly obtaining cell-cell adhesion morphology, the medium (ii) preferably comprises a ROCK inhibitor, a TGFβ receptor inhibitor, a GSK-3β inhibitor, EGF, HGF, a sphingophospholipid, and a glycerophospholipid.

In one aspect, the medium (ii) does not have a composition comprising any given combination of one or more types selected from the group consisting of nicotinamide, ascorbic acid 2-phosphate, dexamethasone, and 10% or more of FBS.

In one aspect, the medium of the present invention is a medium comprising at least 9 types selected from a ROCK inhibitor, a TGFβ receptor inhibitor, a GSK-3β inhibitor, a cAMP signaling activator, EGF, HGF, a sphingophospholipid, a glycerophospholipid, a γ secretase inhibitor, oncostatin M, and dexamethasone (hereinafter, this medium referred to as medium (iii)).

The ROCK inhibitor, TGFβ receptor inhibitor, GSK-3β inhibitor, cAMP signaling activator, EGF, HGF, sphingophospholipid, and glycerophospholipid in the medium (iii) can be selected or determined in the same manners as those as described for the cultures of (i) and (ii). It is to be noted that such selection or determination may be carried out independently from the cultures of (i) and (ii).

The TGFβ receptor inhibitor in the medium (iii) may be selected, as described in the explanation for the method for producing plateable hepatocytes in the present specification. The concentration of the TGFβ receptor inhibitor in the medium (iii) may be, in the case of A83-01, for example, 0.05 to 10 µM, preferably 0.1 to 5 µM, and more preferably 0.5 µM to 3 µM, in the medium. On the other hand, in the case of SB431542, the concentration thereof in the medium (iii) may be, for example, 0.5 to 50 µM, preferably 1 to 30 µM, more preferably 5 to 20 µM, in the medium. When a compound different from A83-01 or SB431542 is used, the concentration thereof in the medium (iii) can be set by those skilled in the art in accordance with the above-described concentration range, taking into consideration the differences between the characteristics of the compound used and those of the A83-01 or the SB431542 (especially, differences in activity). Moreover, whether or not the set concentration range is appropriate can be confirmed by preliminary experiments in accordance with the after-mentioned Examples.

The γ secretase inhibitor in the medium (iii) may be selected, as described in the explanation for the method for producing plateable hepatocytes in the present specification. The concentration of the γ secretase inhibitor (in the case of DAPT) in the medium (iii) may be, for example, 0.1 to 50 µM, preferably 0.5 to 30 µM, and more preferably 0.1 to 20 µM, in the medium. It is to be noted that when a γ secretase inhibitor different from DAPT is used, the concentration thereof in the medium (iii) can be set by those skilled in the art in accordance with the above-described concentration range, taking into consideration the differences between the characteristics of the γ secretase inhibitor used and those of the DAPT (especially, activity). Moreover, whether or not the set concentration range is appropriate can be confirmed by preliminary experiments.

The concentration of the oncostatin M in the medium (iii) may be, for example, 1 to 50 ng/mL, preferably 5 to 40 ng/mL, and more preferably 10 to 30 ng/mL, in the medium. In addition, the concentration of the dexamethasone in the medium (iii) may be, for example, 0.1 to 50 µM, preferably 0.5 to 30 µM, and more preferably 0.1 to 20 µM.

The cAMP signaling activator in the medium (iii) may be one or more selected from the group consisting of a cAMP derivative, a cAMP-degrading enzyme inhibitor, and a cAMP activator, and it is preferably a cAMP activator. The cAMP signaling activator in the medium (iii) may be selected, as described in the explanation for the method for producing plateable hepatocytes in the present specification.

The concentration of the cAMP derivative (in the case of 8-Br-cAMP) in the medium (iii) may be, for example, 0.1 mM to 10 mM, preferably 0.2 mM to 5 mM, and more preferably 0.5 mM to 2 mM, in the medium. It is to be noted that when a cAMP derivative different from 8-Br-cAMP is used, the concentration thereof in the medium can be set by those skilled in the art in accordance with the above-described concentration range, taking into consideration the differences between the characteristics of the cAMP derivative used and those of the 8-Br-cAMP (especially, differences in activity). Moreover, whether or not the set concentration range is appropriate can be confirmed by preliminary experiments.

The concentration of the cAMP-degrading enzyme inhibitor (in the case of IBMX) in the medium (iii) may be, for example, 0.05 mM to 5 mM, preferably 0.1 mM to 3 mM, and more preferably 0.2 mM to 1 mM, in the medium. It is to be noted that when a cAMP-degrading enzyme inhibitor different from IBMX is used, the concentration thereof in the medium (iii) can be set by those skilled in the art in accordance with the above-described concentration range, taking into consideration the differences between the characteristics of the cAMP-degrading enzyme inhibitor used and those of the IBMX (especially, differences in activity). Moreover, whether or not the set concentration range is appropriate can be confirmed by preliminary experiments.

The concentration of the cAMP activator (in the case of forskolin) in the medium (iii) may be, for example, 1 to 50 µM, preferably 3 to 30 µM, and more preferably 5 to 15 µM, in the medium. It is to be noted that when a cAMP activator different from forskolin is used, the concentration thereof in the medium (iii) can be set by those skilled in the art in accordance with the above-described concentration range, taking into consideration the differences between the characteristics of the cAMP activator used and those of the forskolin (especially, differences in activity). Moreover, whether or not the set concentration range is appropriate can be confirmed by preliminary experiments in accordance with the after-mentioned Examples.

In one preferred aspect, the medium (iii) comprises a ROCK inhibitor, a cAMP signaling activator, EGF, HGF, a sphingophospholipid, a glycerophospholipid, a γ secretase inhibitor, oncostatin M, and dexamethasone, or comprises at least 10 types selected from a ROCK inhibitor, a TGFβ receptor inhibitor, a GSK-3β inhibitor, a cAMP signaling activator, EGF, HGF, a sphingophospholipid, a glycerophospholipid, a γ secretase inhibitor, oncostatin M, and dexamethasone. From the viewpoint of quickly obtaining cell-cell adhesion morphology, the medium (iii) preferably comprises a ROCK inhibitor, a TGFβ receptor inhibitor, a GSK-3β inhibitor, a cAMP signaling activator, EGF, HGF, a sphingophospholipid, a glycerophospholipid, a γ secretase inhibitor, oncostatin M, and dexamethasone.

In one aspect, the medium (iii) does not have a composition comprising any given combination of one or more types selected from the group consisting of nicotinamide and ascorbic acid 2-phosphate.

### < Kit >

In one aspect, the present invention relates to a kit comprising the medium of the present invention and suspension hepatocytes. The number of cells of the suspension hepatocytes comprised in the kit of the present invention is not particularly limited, and it may be, for example, 1 x 10⁴ to 1 x 10⁸ cells, 1 x 10⁵ to 1 x 10⁷ cells, etc. The suspension hepatocytes comprised in the kit of the present invention may be frozen.

The kit of the present invention may comprise a vessel containing the medium of the present invention and a vessel containing suspension hepatocytes. These vessels are not particularly limited, as long as they are suitable for preservation of the medium or the cells. Moreover, the kit of the present invention may also comprise an instruction manual containing operational instructions, such as a description of the culture method.

### Examples

Hereinafter, the present invention will be more specifically described in the following examples. However, the present invention is not interpreted, while limiting to these examples.

### < Example 1 >

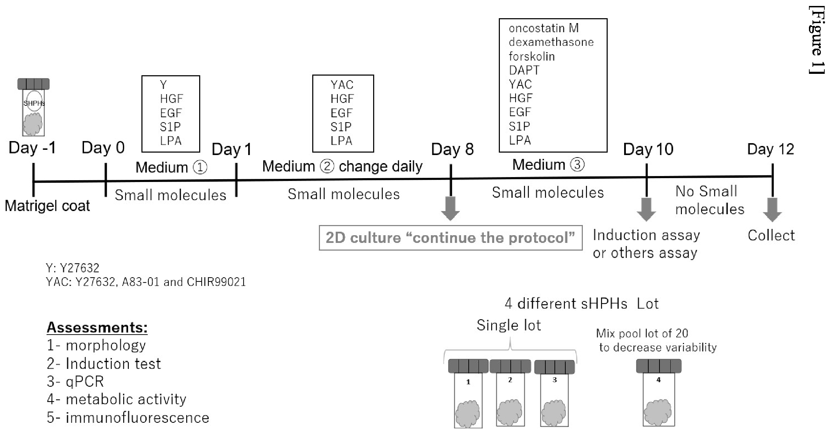

An outline of the experimental protocol of Example 1 is shown in Figure 1.

### [Methods]

### 1. Primary human hepatocytes

Cryopreserved suspension human primary hepatocytes (single donor-derived hepatocytes, and pooled hepatocytes derived from 20-donors) were purchased from Sekisui Xeno Tech.

### 2. Seeding and adhesion conversion HPHs using Medium 1

HPHs were seeded at a density of 3 x 10⁴ cells/cm² on a plate previously coated with Matrigel, using Medium 1. Thereafter, the cells were cultured overnight (3 to 24 hours) at 37°C in a normal oxygen concentration environment (5% CO₂ incubator). It is to be noted that Medium 1 consisted of DMEM/F12 supplemented with 4% fetal bovine serum (FBS), x 1 penicillin-streptomycin solution (x 100) (PS), x 1 GlutaMAX (x 100), x 1 N2 supplement, 10 µg/mL ascorbic acid, 20 ng/mL hepatocyte growth factor (HGF), 20 ng/mL epidermal growth factor (EGF), 1 µM sphingosine monophosphate (S1P) and 5 µM lysophosphatidic acid (LPA), and 10 µM Y27632, and that Medium 1 may or may not comprise an additional 10 µM of Y27632.

### 3. Two-dimensional culture system: (A new culture system for HPHs)

After seeding HPHs, cell adhesion was evaluated, and thereafter, based on lots and cell morphology, the cells were cultured for 7 to 12 days in Medium 2 consisting of DMEM/F12 supplemented with 4% FBS, x 1 PS, x 1 GlutaMAX (x 100), 10 µg/mL ascorbic acid, 20 ng/mL HGF, 20 ng/mL EGF, 1 µM S1P and 5 µM LPA, 1 µM A83-01, 3 µM CHIR99021, and 10 µM Y27632. During this culture period, the culture medium was changed every day. Subsequently, in order to improve the maturation function of the cultured HPHs, Medium 3 that consisted of DMEM/F12 supplemented with 4% FBS, x 1 B27 supplement without vitamin A, x 1 PS, x 1 GlutaMAX (x 100), x 1 N2 supplement, 10 µg/mL ascorbic acid, 20 ng/mL HGF, 20 ng/mL EGF, 1 µM S1P, 5 µM LPA, 1 µM A83-01, 3 µM CHIR99021, 10 µM Y27632, 10 µM DAPT, 20 ng/mL oncostatin M (OSM), 10 µM dexamethasone, 10 µM SB431542, and 10 µM forskolin, was used, and the cells were further cultured for 2 days, and the medium was then changed. SB431542 and forskolin improve the characteristics of HPHs. Furthermore, cell morphology can be improved by adding 0.25% DMSO to Medium 3. Next, mature HPHs could be overlaid with Matrigel and could be subjected to a sandwich culture. On the other hand, such a sandwich culture was not always performed. Finally, the cells were washed with a phosphate-buffered saline (PBS) and were then used for drug metabolic enzyme induction assays and other assays.

### Assay

### 4. Imaging using phase-contrast microscope

The morphology of HPHs was observed and evaluated every day using a phase-contrast microscope. The images of HPHs were recorded before and after the medium change on Day 0, and before and after the induction assay on Day 12.

### 5. Cytochrome 450 (CYP) induction assay and mRNA expression

After the morphology of HPHs had been confirmed on Day 10 after the start of culture, omeprazole, phenobarbital, or rifampicin, which are known as inducers of CYP1A2, CYP2B6, or CYP3A4, respectively, were added to a commercially available opti incubate medium. These were added to final concentrations of 50 µM, 750 µM, or 25 µM, respectively. 6-(4-Chlorophenyl)imidazo[2,1-b][1,3]thiazole-5-carbaldehyde O-(3,4-dichlorobenzyl)oxime, an inducer of CYP2B6, was added to a final concentration of 1 nM. As a control, a medium containing less than 0.1% DMSO was used, and the cells were incubated for 48 hours. Each CYP induction medium was newly prepared and replaced every day. Thereafter, the cells were harvested and were then subjected to quantitative real-time PCR (qPCR).

### 6. CYP metabolic activity after induction assay

After the morphology of the cultured HPHs had been confirmed on Day 10, 50 µM omeprazole, 750 µM phenobarbital, or 25 µM rifampicin was each added to a commercially available opti incubate medium, and the induction assay was carried out for CYP1A2, CYP2B6, or CYP3A4. A medium containing less than 0.1% DMSO was used as a control, and the cells were incubated for 48 hours. Each CYP induction medium was newly prepared and replaced every day, and thereafter, the cells were harvested. Thereafter, on Day 12, a CYP activity assay was performed using a CYP substrate cocktail as follows. That is, the cells were incubated in a substrate-supplemented medium at 37°C for 30 minutes to 4 hours. 50 µM phenacetin was used as a substrate for CYP1A2, 500 µM bupropion for CYP2B6, 5 µM diclofenac for CYP2C9, 50 µM *S*-mephenytoin for CYP2C19, and 5 µM midazolam for CYP3A4. Thereafter, the culture supernatants were recovered, and metabolites were quantified by LC-MS/MS.

### [Results]

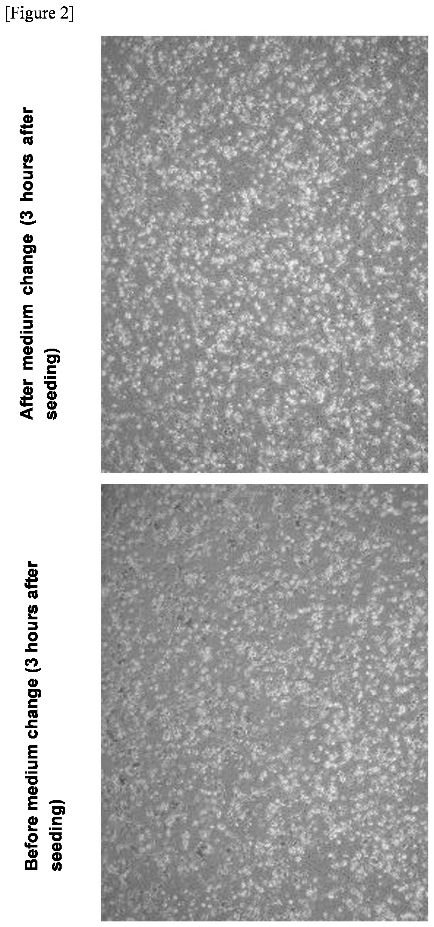

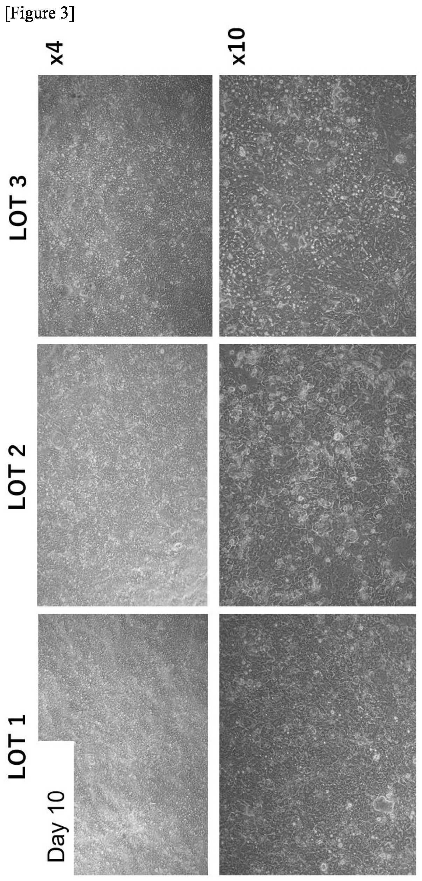

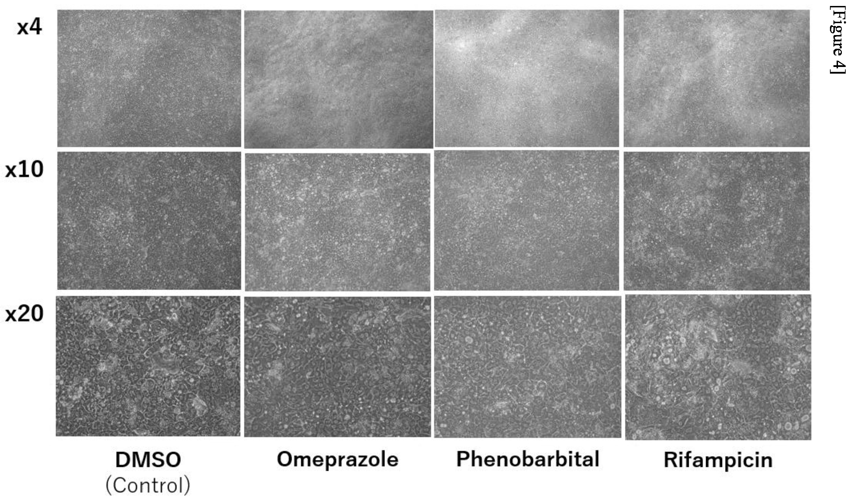

The morphological images of HPHs from Lot 1 before and after medium change on Day 0 (Figure 2) showed that the cells were adhered to the plate. Furthermore, the morphological images of HPHs from each of the three lots in a two-dimensional culture system (without passaging, before induction assay) on Day 10 (Figure 3) also showed that the cells were plateable. The morphological images of HPHs from Lot 1 in a two-dimensional culture system (without passaging) after the induction assay are shown in Figure 4. From these results, it was demonstrated that the HPHs obtained by the method of the present invention can be cultured two-dimensionally as plateable cells, even if they are suspension-type cells.

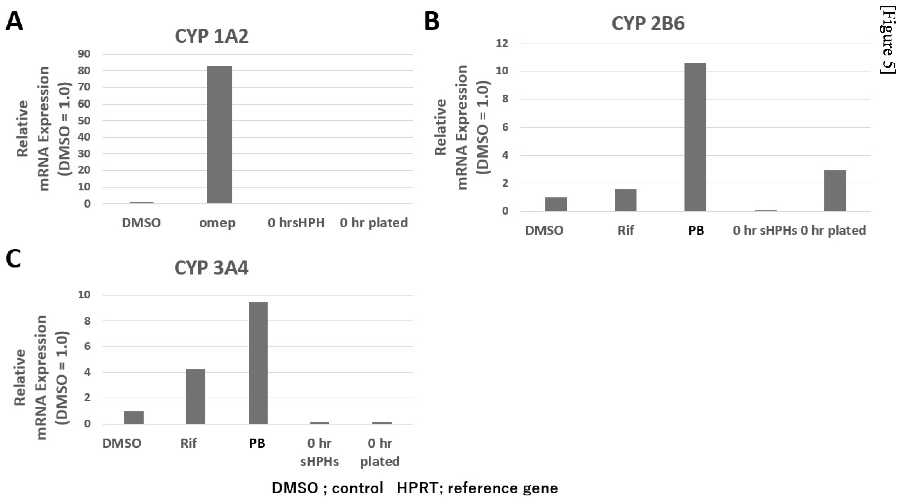

The results of measuring the mRNA expression of each metabolic enzyme in the induction assay are shown in Figure 5. CYP1A2 mRNA expression was induced about 80 times by omeprazole (Figure 5A). CYP2B6 mRNA expression was induced by phenobarbital, but not by rifampicin (Figure 5B). CYP3A4 mRNA expression was induced about 4 times and about 8 times, respectively, by rifampicin and phenobarbital (Figure 5C). From these results, it was demonstrated that the HPHs obtained by the method of the present invention can be used in induction assays.

### < Example 2 >

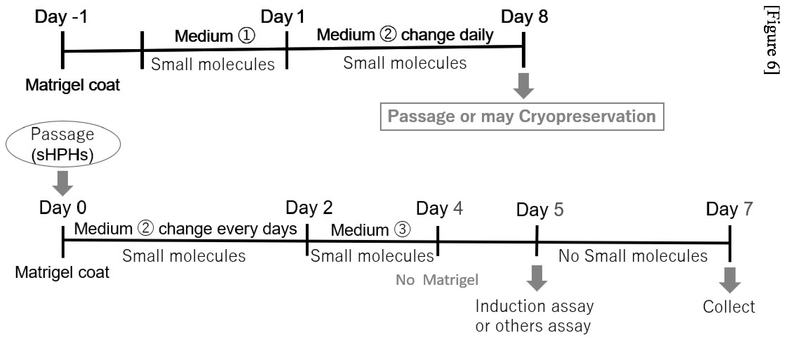

An outline of the experimental protocol of Example 2 is shown in Figure 6.

### [Methods]

### 7. Passage culture, CYP induction assay and mRNA expression HPHs

In order to verify whether HPHs could be passaged *in vitro* as plateable HPHs, the cells were cultured for 6 to 8 days for every lot, the morphology thereof was then confirmed, and thereafter, the cells were harvested using commercially available Trypsin-EDTA (TE) as a cell dissociation solution. The passaged sHPHs were harvested using 0.25% TE. Thereafter, these cells were re-seeded on plates or were cryopreserved. In the case of re-seeding, the cells were re-seed at a density of 3 x 10⁴ cells/cm² on plates that had been previously coated with Matrigel. Medium 2 was used for daily changes of the medium. After the morphology had been confirmed, the cells were further cultured for another 2 days using Medium 3 without overlaying with Matrigel, followed by medium change. On Day 5, induction assays for CYP1A2, CYP2B6, and CYP3A4 were performed using the same method as that in Example 1. Cell morphology was confirmed on Day 7 during the induction assay. Finally, the cultured HPHs were harvested and were then subjected to quantitative real-time PCR (qPCR). Cell cryopreservation was performed by suspending cells in a commercially available cell cryopreserving solution, Stem Cell Banker or Cell Banker plus 1, according to the instruction manual.

### 8. CYP metabolic activity after induction assay

After the morphology of the cultured HPHs had been confirmed on Day 7, a CYP metabolic activity assay was performed. The culture supernatants were recovered, and metabolites were then quantified by LC-MS/MS using the same method as that in Example 1.

### [Results]

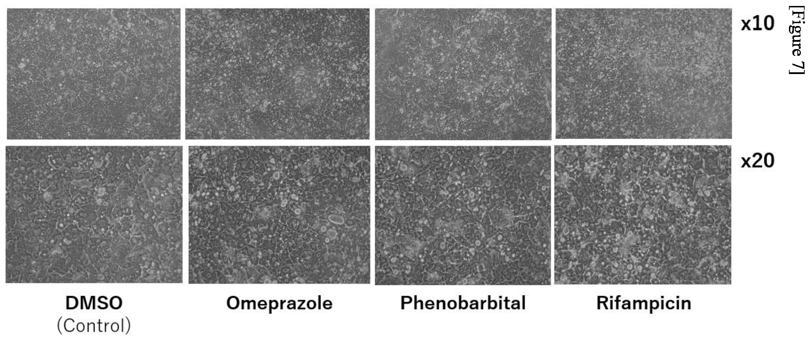

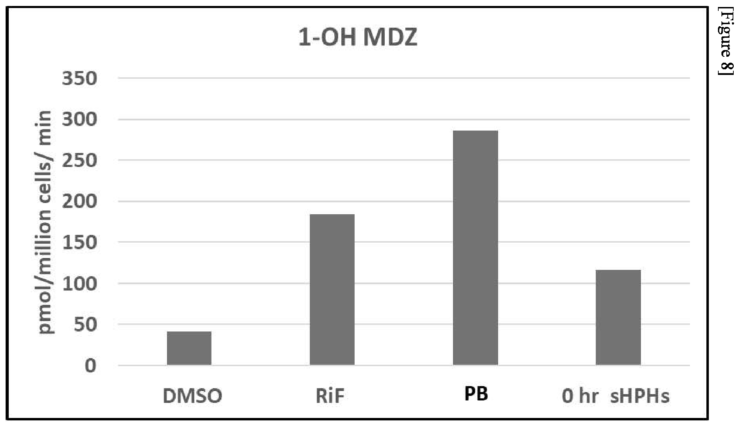

From the morphological images (Figure 7) of Lot 1 (after induction assay, with passage, without Matrigel overlaying) on Day 7, it was demonstrated that the HPHs obtained by the method of the present invention can be passaged. Furthermore, while it had previously been impossible to maintain HPHs in a living state for 4 to 5 hours or more, it was demonstrated that this was possible with the HPHs obtained by the method of the present invention. In addition, from the results of measuring the metabolic activity of CYP3A4 using these cells (Figure 8), it was demonstrated that induction assays and metabolic activity assays are possible even after passage, in the case of using the plateable HPHs obtained by the method of the present invention.

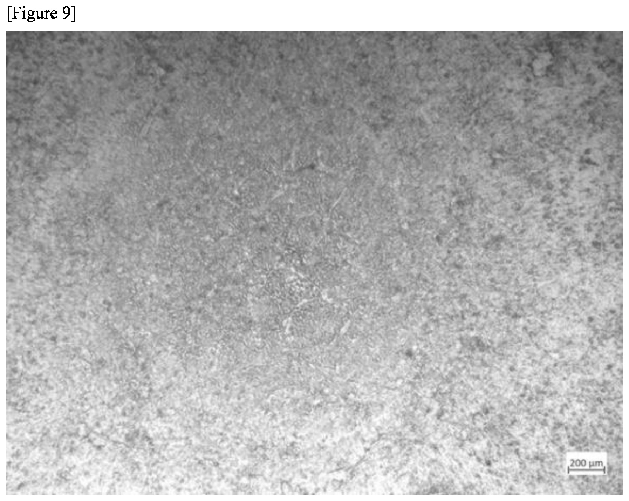

For cryopreservation, the cells were cultured for 6 to 8 days for every lot, and after confirming the morphology, and the cells were then harvested using the commercially available cell dissociation solution described in the [Methods] section. The harvested HPHs were cryopreserved. Subsequently, the cryopreserved HPHs were cultured for 2 days using Medium 2, and were then re-seed onto a plate that had been previously coated with Matrigel. After confirming the morphology, the cells were changed to Medium 3, and were then cultured for 2 days. The resulting cells were overlaid with 30 times diluted Matrigel, and the morphological images were then confirmed using a phase-contrast microscope. The results are shown in Figure 9.

### < Example 3 >

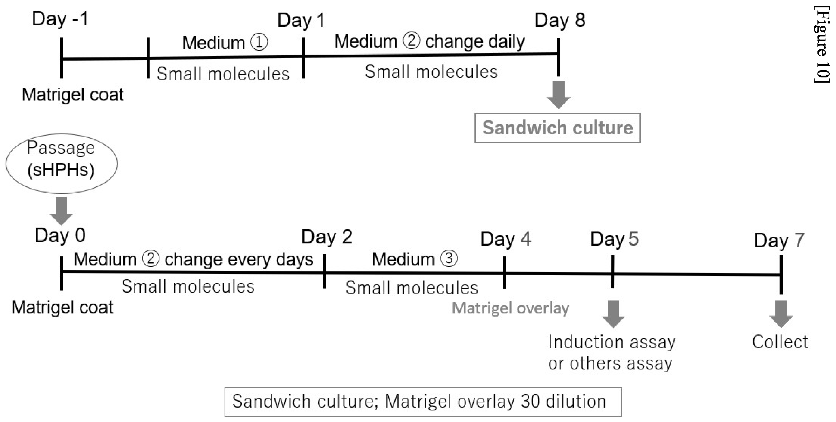

An outline of the experimental protocol of Example 3 is shown in Figure 10.

### [Methods]

### 9. Sandwich culture, CYP induction assay and mRNA expression measurement of passaged HPHs

In order to verify whether HPHs can be sandwich-cultured as plateable HPHs, trypsin-treated HPHs were seeded on a plate that had been coated with Matrigel. The cells were cultured in Medium 2 for 2 days, and the medium was changed every day. Thereafter, the morphology was confirmed, and the cells were cultured for another 2 days in Medium 3 changed from Medium 2. On Day 4, the cells were overlaid with 30-fold diluted Matrigel, and were then incubated in a normal oxygen concentration environment (5% CO₂ incubator) to perform a sandwich culture. On Day 5, induction assays and other assays were performed using the sandwich-cultured HPHs. Induction assays for CYP1A2, CYP2B6, and CYP3A4 were performed using the same method as those in Examples 1 and 2, and the cell morphology after the induction assays was confirmed by imaging. Finally, the cultured HPHs were harvested, and were then subjected to quantitative real-time PCR (qPCR).

### 10. CYP metabolic activity after induction assay

On Day 7, after confirming the morphology of HPHs, a CYP metabolic activity assay was performed. The culture supernatants were recovered, and metabolites were quantified by LC-MS/MS, as in Example 2.

### [Results]

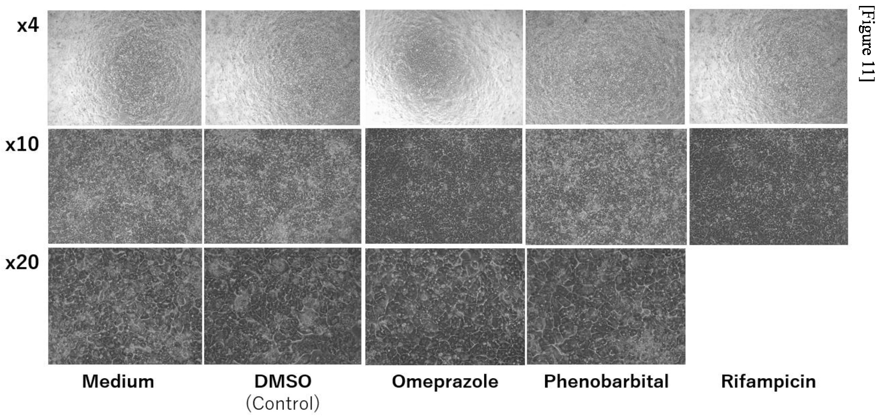

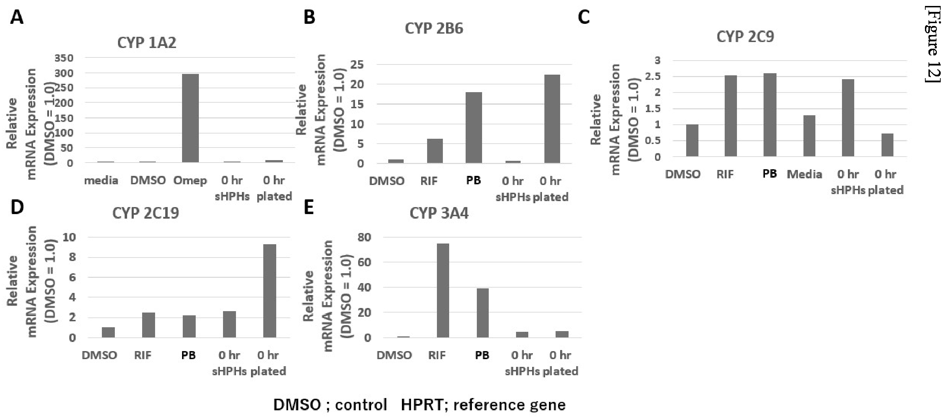

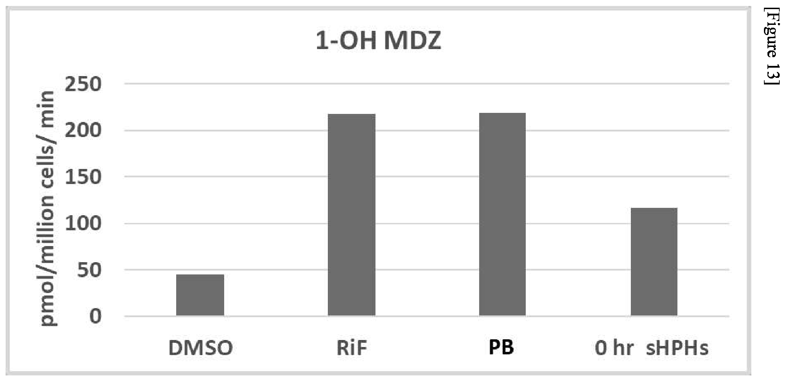

The morphological images of the sandwich-cultured HPHs from Lot 1 (after induction assay, with passage) on Day 7 are shown in Figure 11. In addition, the results of measuring the mRNA expression of each metabolic enzyme in the induction assay using the sandwich-cultured HPHs from Lot 1 (after induction assay, with passage) on Day 7 are shown in Figure 12. CYP1A2 mRNA expression was induced about 300 times by Omeprazole to (Figure 12A). CYP2B6 mRNA expression was induced about 5 times and about 17 times by rifampicin and phenobarbital, respectively (Figure 12B). CYP2C9 mRNA expression was induced about 2.5 times by both rifampicin and phenobarbital (Figure 12C). CYP2C19 mRNA expression was induced about 2.5 times by both rifampicin and phenobarbital (Figure 12D). CYP3A4 mRNA expression was induced about 70 times and about 40 times by rifampicin and phenobarbital, respectively (Figure 12E). Furthermore, the results of measuring CYP3A4 metabolic activity using the sandwich-cultured HPHs from Lot 1 (after induction assay, with passage) on Day 7 are shown in Figure 13.

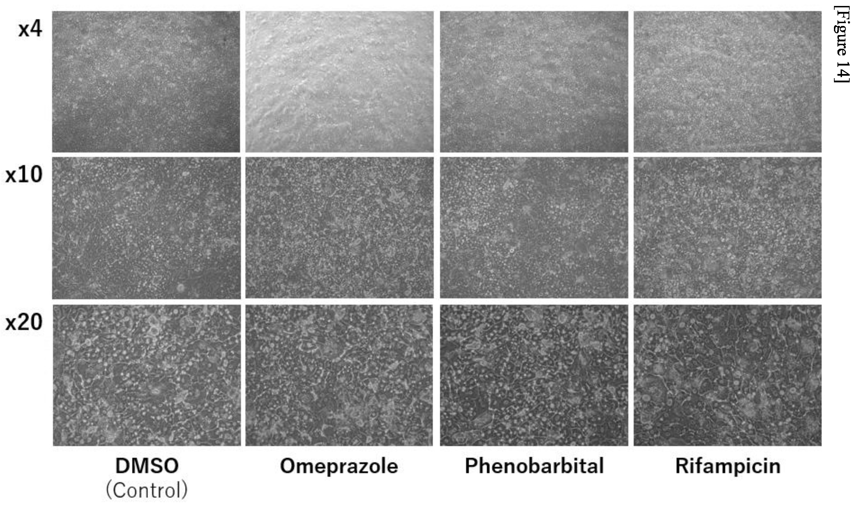

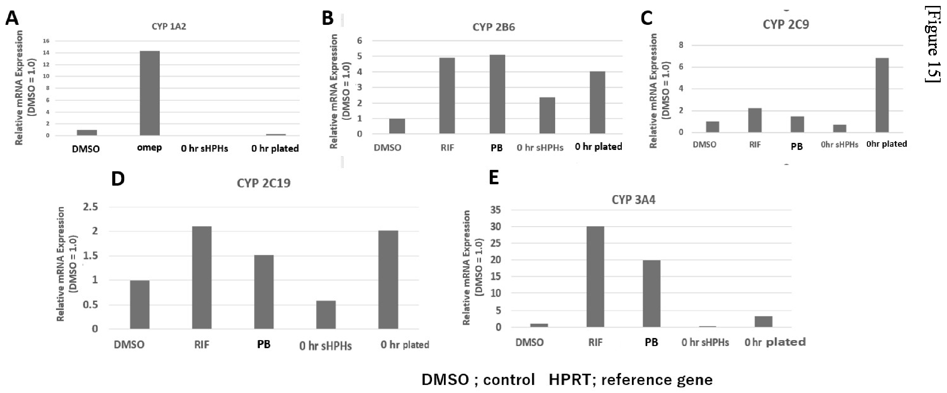

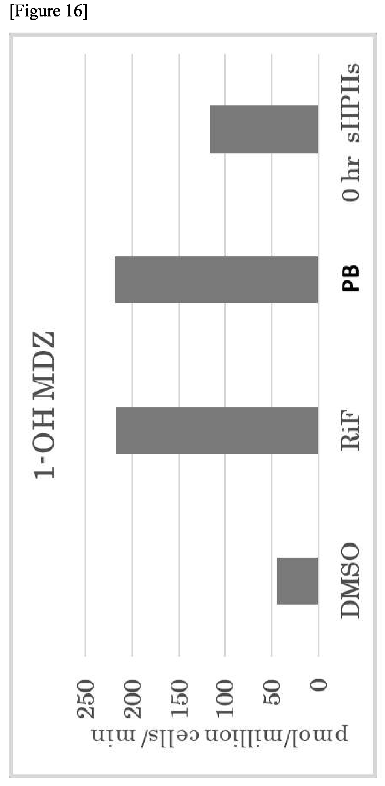

The morphological images of the sandwich-cultured HPHs from Lot 2 (after induction assay, with passage) on Day 7 are shown in Figure 14. In addition, the results of measuring the mRNA expression of each metabolic enzyme in the induction assay using the sandwich-cultured HPHs from Lot 2 (after induction assay, with passage) on Day 7 are shown in Figure 15. CYP1A2 mRNA expression was induced about 14 times by omeprazole to (Figure 15A). CYP2B6 mRNA expression was induced about 5 times by both rifampicin and phenobarbital (Figure 15B). CYP2C9 mRNA expression was induced about 2 times or more by rifampicin, but was not induced by phenobarbital (Figure 15C). CYP2C19 mRNA expression was induced about 2 times and about 1.5 times by rifampicin and phenobarbital, respectively (Figure 15D). CYP3A4 mRNA expression was induced about 30 times and about 20 times by rifampicin and phenobarbital, respectively (Figure 15E). Furthermore, the results of measuring CYP3A4 metabolic activity using the sandwich-cultured HPHs from Lot 2 (after induction assay, with passage) on Day 7 are shown in Figure 16.

From the aforementioned results, it was demonstrated that the HPHs obtained by the method of the present invention can be sandwich-cultured. Furthermore, it was demonstrated that induction assays or CYP metabolic activity assays can be performed when the plateable HPHs obtained by the method of the present invention are sandwich-cultured.

### < Example 4 >

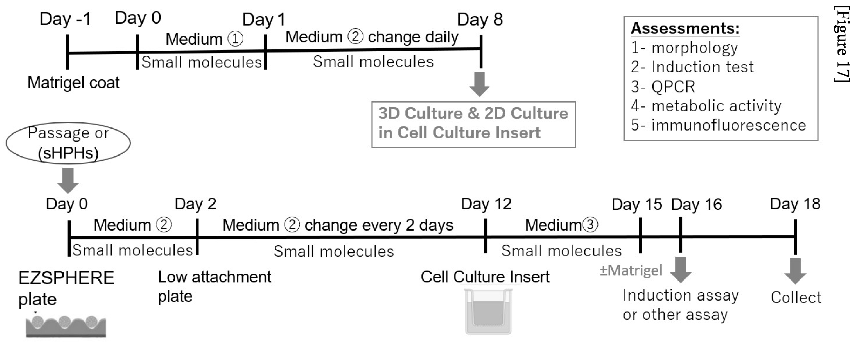

An outline of the experimental protocol of Example 4 is shown in Figure 17.

### [Methods]

### 11. Three-dimensional culture system, and two-dimensional culture system on cell culture inserts , of HPHs

Each lot was cultured for 6 to 8 days, and after confirming morphology, the cells were harvested using 0.25% TE. Thereafter, the cells were seeded on EZSPHERE plates and were cultured for 1 or 2 days to create uniform spheres. After the sphere formation, the cells were subjected to a suspension culture in Medium 2 containing 30-fold diluted Matrigel for 6 to 12 days based on the lot and the morphology. Thereafter, the cells were dissociated into single cells using TrypLE Select^{™}. Subsequently, the cells were cryopreserved or re-seeded. Cryopreservation was performed by suspending in a commercially available cryopreserve solution, as described in Example 2. In the case of re-seeding, the cells were cultured in Medium 2 on Matrigel-pre-coated cell culture inserts for about 2 days. Thereafter, the morphology was confirmed, and the medium was changed. In order to promote the maturation of HPHs, the cells were cultured for an additional 2 days using Medium 3. The resulting cells were overlaid with Matrigel. Subsequently, the cells were washed with PBS, and were then used in induction assays and other assays.

### 12. CYP induction assay and measurement of mRNA expression

On Day 18, based on the cell morphology, mRNA expression was evaluated by the same method as that of Example 3.

### 13. CYP metabolic activity after induction assay

On Day 18, based on the cell morphology, metabolic activity was evaluated by the same method as that of Example 3.

### 14. Immunofluorescence Staining

The cultured HPHs cells were fixed and permeabilized with cold 100% methanol at room temperature for 5 minutes. Thereafter, the cells were washed with PBS. The cells were blocked with 5% fetal bovine serum (FBS) at room temperature for 20 minutes. Thereafter, the cells were treated with a primary antibody at 4°C overnight. The following day, the cells were rinsed with PBS and were then incubated with a secondary antibody at room temperature for 1 hour. Subsequently, the nuclei were stained with DAPI and were then observed under a KEYENCE fluorescence microscope.

### [Results]

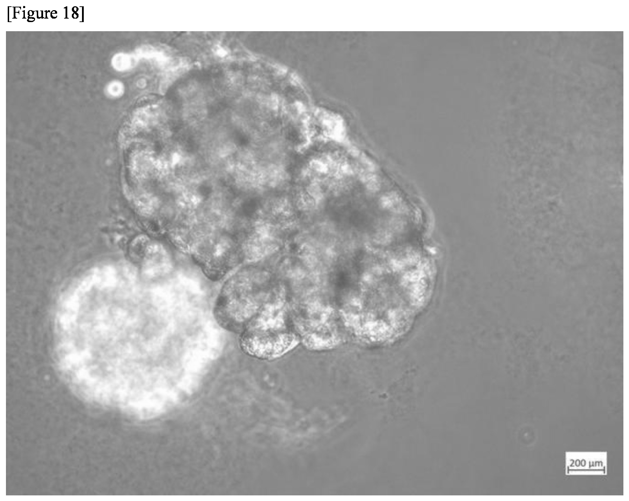

From the morphological image on Day 20 of the culture (Figure 18), it was demonstrated that three-dimensional spheroids can be produced using the HPHs obtained by the method of the present invention.

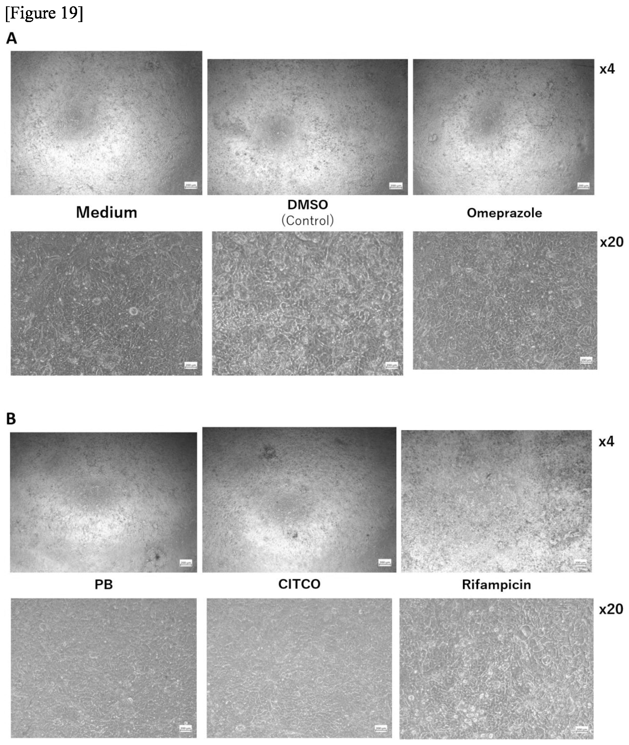

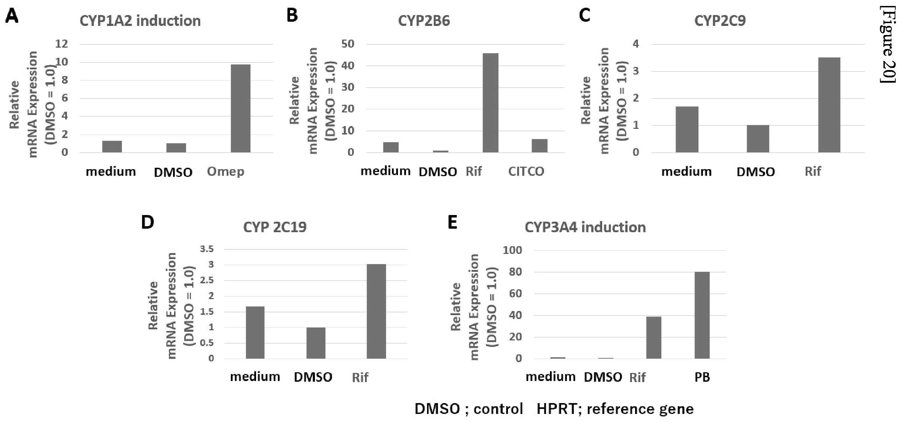

The morphological images of a two-dimensional culture (Lot 2, after induction assay) on cell culture inserts on Day 18 are shown in Figures 19A and B. In addition, the results of measuring the mRNA expression of each metabolic enzyme in an induction assay using a two-dimensional culture (Lot 2, after induction assay) on cell culture inserts on Day 18 are shown in Figure 20. It was demonstrated that three-dimensional spheroids can be produced from the HPHs obtained by the method of the present invention, and that a two-dimensional culture can be carried out thereafter. While the conventional culture period for primary hepatocytes was approximately 4 or 5 days, I was demonstrated that the HPHs obtained by the method of the present invention can be cultured for a long period of time of about 1 month, and that induction assays and other assays can be carried out even when the HPHs are further cultured for a longer period of time.

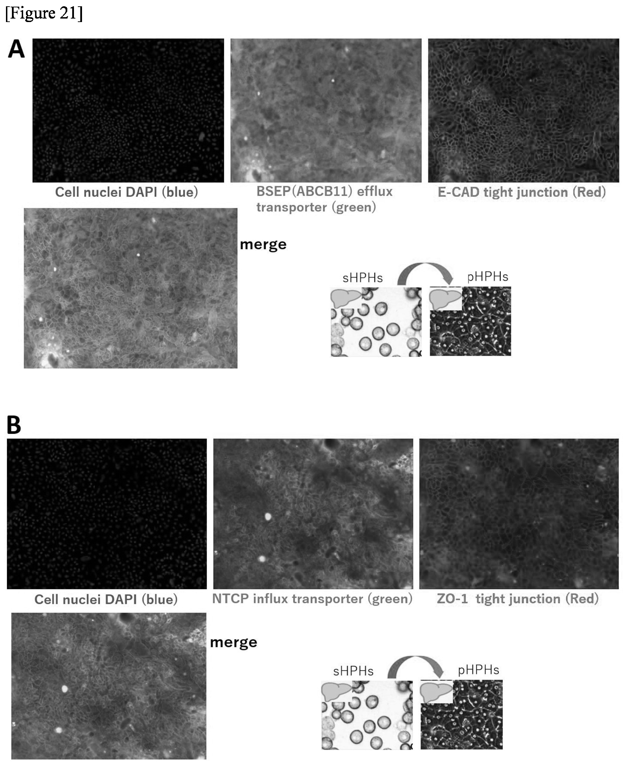

The results of the immunofluorescence staining of tight junctions and transporters in cells on Day 18, in which the spheroids were two-dimensionally cultured are shown in Figures 21A and B. In cells in which spheroids were produced from the plateable HPHs obtained by the present invention and were then two-dimensionally cultured, cell adhesion was observed. Moreover, the protein expression of the tight junctions and the transporters was observed in these cells.

### < Example 5 >

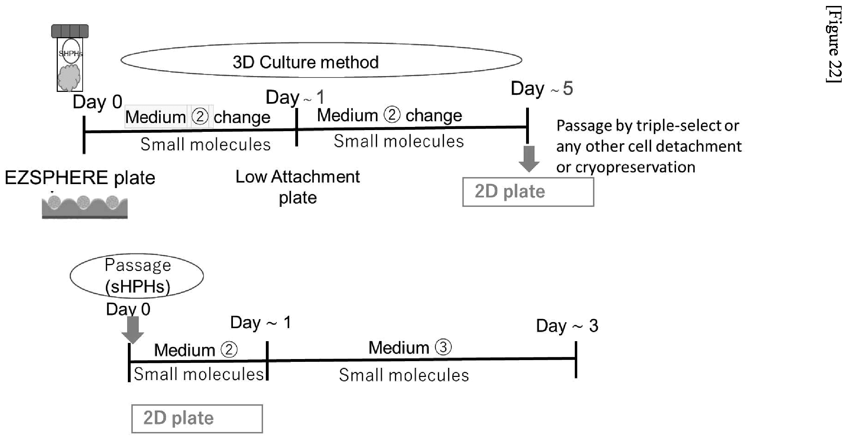

An outline of the experimental protocol of Example 5 is shown in Figure 22. Whether spheroids created from suspension HPHs by a suspension culture could be passaged *in vitro* as plateable HPHs was verified. In addition, whether this could shorten culture time and improve function was also examined.

### [Methods]

### 15. Culture of suspension human primary hepatocytes (sHPHs)

A single lot of cryopreserved suspension human primary hepatocytes (sHPHs) was plated onto a EZSPHERE^{®} plate, and homogeneous spheres were created based on cell morphology at a density of 3 x 10⁴ cells/cm² in Medium 2 for 1 or 2 days. Subsequently, the cells were incubated at 37°C under normal oxygen conditions (5% CO₂ incubator). After the sphere formation, the cells were cultured for a further 4 days as a suspension culture in 30-fold diluted Matrigel-containing Medium 2 on a low-adhesion plate. Thereafter, the cells were harvested a tube and were then washed with PBS (-). TrypLE Select^{™} was added, and the cells were incubated at 37°C for 15 minutes and were then confirmed again. After formation of single cells, a 10% FBS-containing medium was added, and the cells were then centrifuged at 1000 rpm for about 3 minutes. Thereafter, cryopreservation or re-seeding was performed. In the case of re-seeding, passaged sHPHs were plated onto a Transwell plate that had been pre-coated with Matrigel, and were then cultured in Medium 2 for about 1 day. Then, morphology was confirmed, the medium was then changed to Medium 3, and the cells were then cultured for another 2 days. The resulting cells were either overlaid with Matrigel (30-fold diluted) and were then subjected to a sandwich culture for 1 day, or were cultured without sandwich culture. After that, the cells were washed with PBS, and were then used for qPCR assays. In the case of cryopreservation, the cells were suspended in a commercially available cryopreserve solution according to the manufacturer's instruction manual.

### 16. Phase-contrast microscopy imaging

The morphological images of sHPHs were obtained using a ZEISS Primovert inverted microscope (Carl ZEISS, Oberkochen, Germany).

### 17. qPCR

Total RNA was extracted using the RNeasy Mini Kit and QIAshredder (QIAGEN, Venlo, Netherlands) according to the manufacturer's instruction manual. RNA concentration was calculated by measuring the absorbance at 260 nm using Nanodrop (Thermo Fisher Scientific). Next, cDNA was prepared from the extracted RNA, using ReverTra Ace qPCR RT Master Mix with gDNA Remover, according to the manufacturer's instructions. Real-time PCR was performed using KAPA SYBR Fast qPCR Kit Master Mix (2X) ABI Prism. The sample was reacted using the Light Cycler 96 System (Roche, Basel, Switzerland). After pre-incubation at 95°C for 3 minutes, in the qPCR process, 40 cycles of 10 seconds at 95°C, 20 seconds at 60°C, and 1 second at 72°C were carried out. Hypoxanthine phosphoribosyltransferase was used as a housekeeping gene, and the real-time PCR results were calculated using ΔΔCt.

### 18. Fluorescence efflux assay (CDFDA assay)

CDFDA (carboxy-DCFDA(5-(and-6)-carboxy-2',7'-dichlorofluorescein diacetate), C369, Invitrogen) is cleaved by intracellular esterases to become fluorescent (CDF) 5-(and-6)-carboxy-2',7'-dichlorofluorescein, which is excreted into the bile duct by the MRP2 efflux transporter. The cells were treated with 10 µM CDFDA for 15 minutes. After this treatment, the cells were washed twice with a Hank's equilibrium salt solution (HBSS), and were then incubated with HBSS for 15 minutes. After the incubation, the cells were further washed twice with HBSS, and finally, HBSS was newly added thereto. The accumulation of CDF (green) in the bile duct was observed using the fluorescence microscope LEICA DMi8 (Leica Microsystem GmbH, Germany).

### [Results]

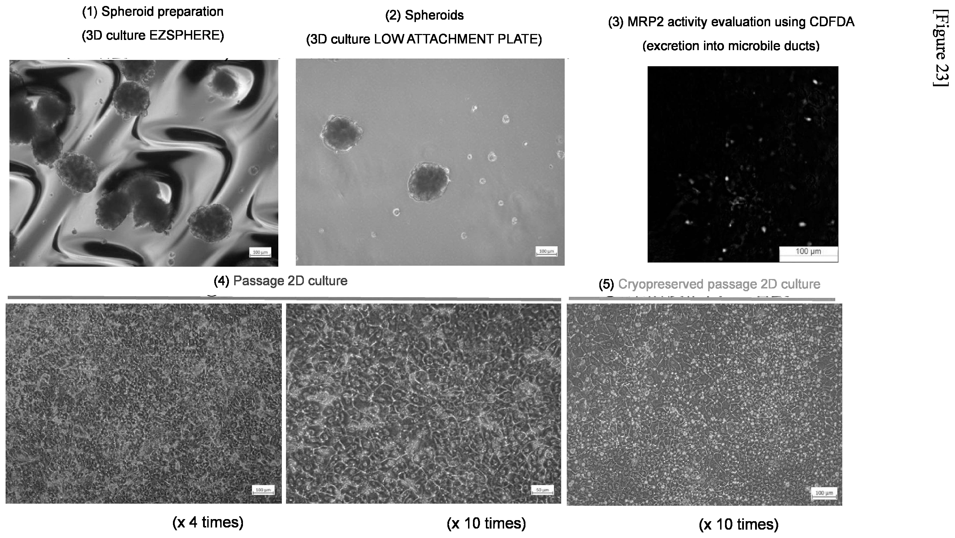

The results of cell morphology (three-dimensional culture, cryopreservation, and passage culture) and CDFDA assay are shown in Figure 23. It became clear that according to the method of the present example, the operation using Medium 1 can be reduced, and that the culture period can be shortened. Moreover, it was elucidated that spheroid formation was possible directly from suspension HPHs (1 and 2 in Figure 23), and that cryopreservation after conversion into single cells was also possible (5 in Figure 23). Furthermore, it became clear from confirmation of drug excretion into the bile canaliculi by MRP2 (3 in Figure 23) that the cells obtained in the present example can be used to test for cholestatic liver injury.

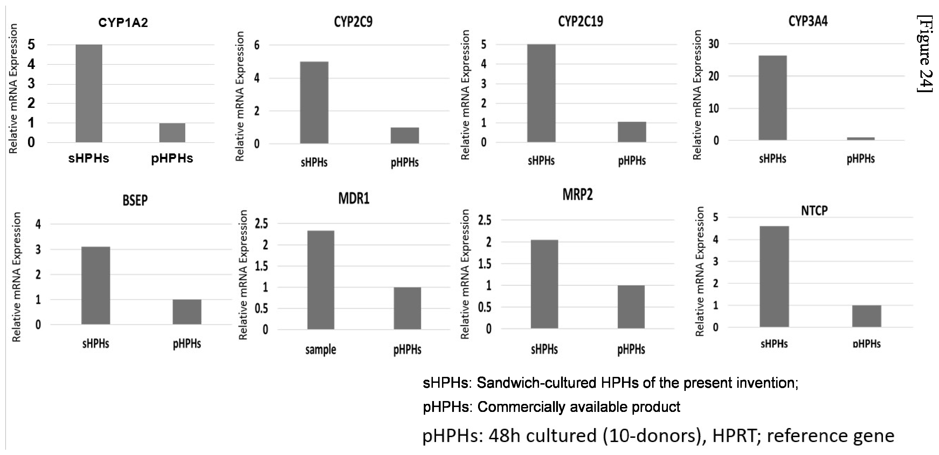

The results of analyzing the mRNA expression of each metabolic enzyme and drug transporter in the sandwich-cultured HPHs on Day 3 after the passage operation are shown in Figure 24. The mRNA expression of each CYP and transporter was significantly higher compared to the HPHs sold as plateable HPHs (shown as pHPHs in Figure 24) that had been cultured for 48 hours.

### < Example 6 >

### [Methods]

### 19. Two-dimensional culture system: (A two-dimensional culture system for sHPHs was used to investigate the factors.)

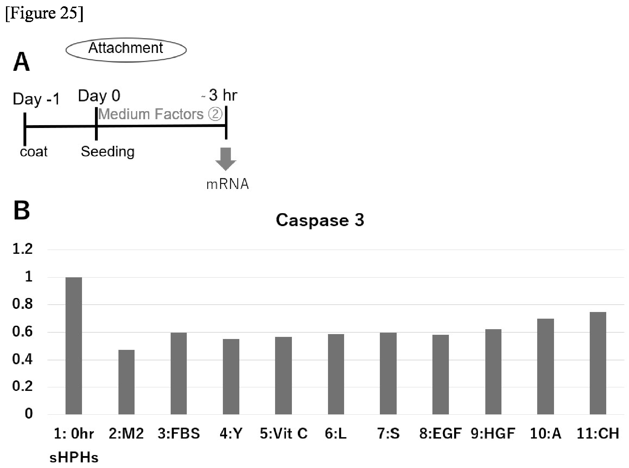

Matrix two-dimensional Transwell plates were divided into a group using Medium 2 and groups, to which only one factor from those contained in Medium 2 was added, and were then pre-coated with Matrigel. Thereafter, sHPHs were seeded at 3 x 10⁴ cells/cm² on Transwell plates. Thereafter, the cells were cultured at 37°C under normal oxygen conditions (5% CO₂ incubator) for 3 hours. Next, mRNA expression was evaluated using the method described above, and the expression of caspase 3, which has an anti-apoptotic action and can promote the longevity, adhesiveness, and culture of sHPHs, was examined (Figure 25A). While all of these factors may directly or indirectly influence on apoptosis and adhesiveness, sHPHs were not investigated in this study.

The Transwell plates were coated with Matrigel, and were then divided into the following 11 groups (n = 1) (Figure 25B).
1: 0 hr sHPHs: sHPHs were thawed and directly collected as a 0 hr control.
2: M2: Medium 2 containing factors highly likely to be involved in adhesion, apoptosis, and culture (FBS, Y27632, vitamin C, lysophosphatidic acid, D-erythro-sphingosine-1-phosphate, EGF, HGF, A83-01, and CHIR99021) was used.
3: Only FBS was added.
4: Only Y27632 was added.
5: Only vitamin C was added.
6: Only lysophosphatidic acid was added.
7: Only D-erythro-sphingosine-1-phosphate was added.
8: Only EGF was added.
9: Only HGF was added.
10: Only A83-01 was added.
11: Only CHIR99021 was added.

Thereafter, the sHPHs were recovered, and qPCR was performed to measure the gene expression of caspase 3.

### [Results]

The results are shown in Figure 25B. It was demonstrated that all factors reduced caspase 3 compared to sHPHs at 0 hours, namely, all factors reduced apoptosis and potentially promoted the lifespan, adhesiveness, and culture of sHPHs.

### < Example 7 >

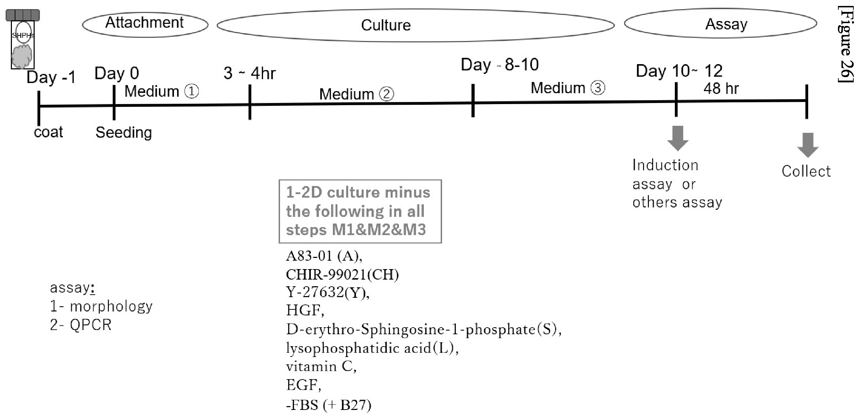

An outline of the experimental protocol of Example 7 is shown in Figure 26.

### [Methods]

### 20. Two-dimensional culture system: (A two-dimensional culture system for sHPHs was used to investigate the factors.)

sHPHs were cultured using an ordinary protocol consisting of 3 steps. Briefly, sHPHs were seeded at 3 x 10⁴ cells/cm² on a matrix two-dimensional Transwell plate pre-coated with Matrigel, and were then cultured for 3 hours. Next, Medium 1 was changed to Medium 2, and the cells were then cultured for 8 days. Then, Medium 2 was changed to Medium 3, and the cells were then cultured for further 2 days. Other groups were also treated by the same ordinary protocol as that described above, but factors were removed from all steps of Medium 1, Medium 2, and Medium 3, as described below (Figure 26). Thereafter, all factors were removed, and the cells were cultured for 1 day and were then subjected to a sandwich culture using 30-fold diluted Matrigel. After that, the cells were washed with a phosphate-buffered saline (PBS), and were prepared for mRNA expression evaluation as described above, and the expression of albumin, CYP1A2, and CYP3A4 was examined.

It is to be noted that the ordinary protocol is a protocol consisting of 3 steps, in which wells are coated with Matrigel, the cells are seeded using Medium 1, 3 to 4 hours later, Medium 2 is added, and then Medium 3 is added on Day 8 and is then used until Day 10.
Medium 1: Components (FBS, Y27632, vitamin C, lysophosphatidic acid (L), D-erythro-sphingosine-1-phosphate (S), EGF, and HGF)
Medium 2: (FBS, Y27632, vitamin C, lysophosphatidic acid (L), D-erythro-sphingosine-1-phosphate (S), EGF and HGF, A83-01, and CHIR99021)
Medium 3: (FBS, Y27632, vitamin C, lysophosphatidic acid (L), D-Erythro-sphingosine-1-phosphate (S), EGF and HGF, A83-01, CHIR99021, DAPT, dexamethasone, OSM, B27, forskolin, and SB431542)
   1. Ordinary protocol
   2. AC (only A83-01 and CHIR99021 were not used in all steps from the ordinary protocol)
   3. Y (only Y27632 is not used in all steps from the ordinary protocol)
   4. A (only A83-01 is not used in all steps from the ordinary protocol)
   5. CH (only CHIR99021 is not used in all steps from the ordinary protocol)
   6. HGF (only HGF is not used in all steps from the ordinary protocol)
   7. S (only D-erythro-sphingosine-1-phosphate is not used in all steps from the ordinary protocol)
   8. L (only lysophosphatidic acid is not used in all steps from the ordinary protocol)
   9. VitC (only vitamin C is not used in all steps from the ordinary protocol)
   10. EGF (only EGF is not used in all steps from the ordinary protocol)
   11. FBS (only FBS is not used in all steps from the ordinary protocol)
   12. (-FBS)+B27 (FBS is not used from the ordinary protocol, and only B27 with vitamin A removed is added as a serum replacement)
   13. iMatrix matrix (ordinary protocol) (laminin (to 60%), one of the four major basement membrane ECM proteins that are the main components of Matrigel)
   14. Collagen matrix (ordinary protocol)

Thereafter, on Day 11, sHPHs were harvested, and qPCR was performed to measure the gene expression of albumin, CYP1A2, and CYP3A4.

### 21. Phase-contrast microscopy imaging

The morphological images of sHPHs were obtained using a ZEISS Primovert inverted microscope (Carl ZEISS, Oberkochen, Germany).

### [Results]

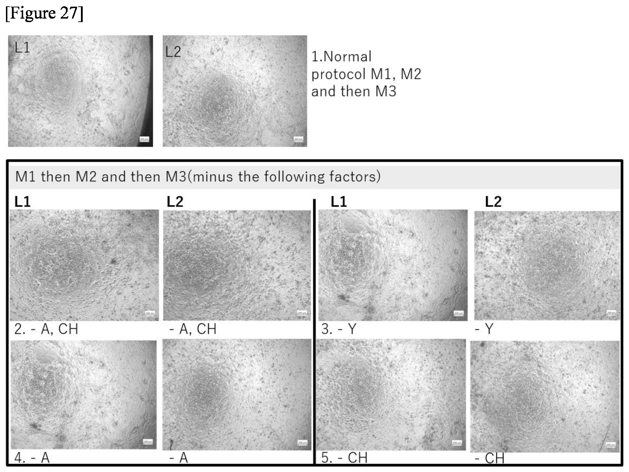

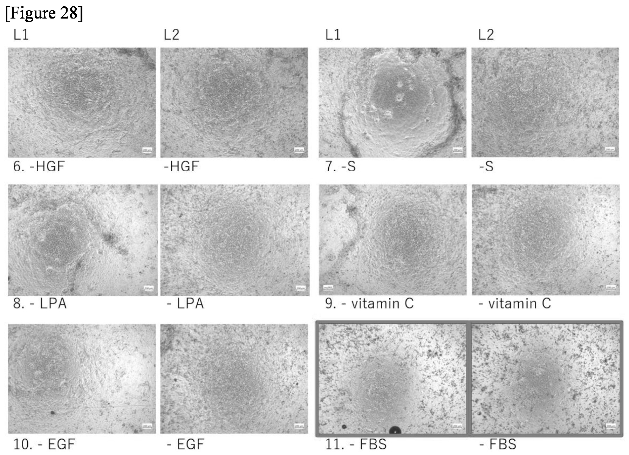

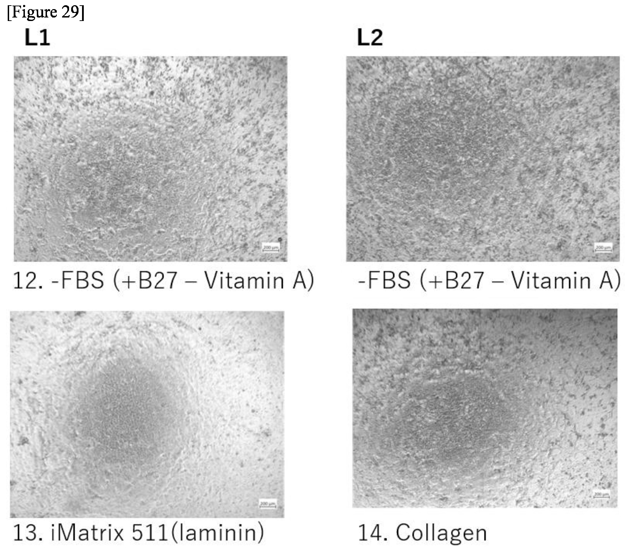

In the ordinary protocol using Medium 1 and Medium 2, cell-cell adhesion morphology was obtained more quickly than the case of not using some factors (Figures 27 to 29). While serum or a serum replacement (FBS, KSR, or B27-vitamin A) is essential for culturing sHPHs, pHPHs could be cultured without such serum (Figure 28). Laminin was found to be one of the Matrigel components usable for culturing and adhering sHPHs (Figure 29). Matrigel and laminin 511 were found to be superior to collagen.

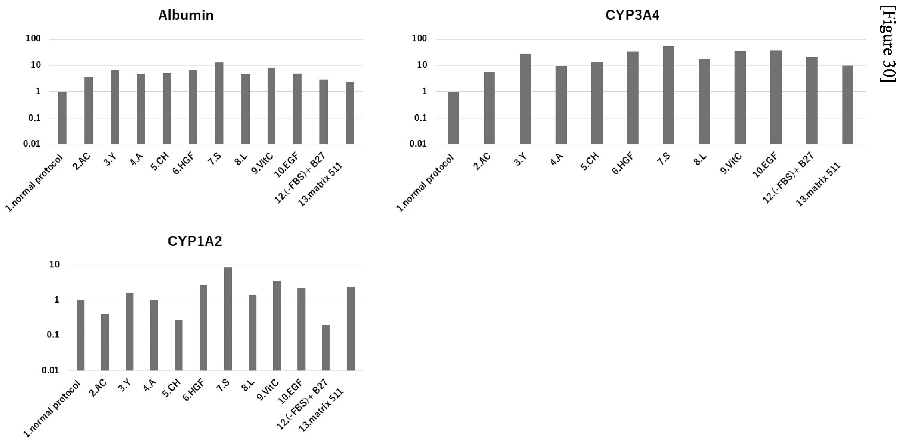

qPCR was performed after culturing in Medium 3 (Figure 30). Culturing in Medium 3 is likely to be necessary for further cell maturation.

### < Example 8 >

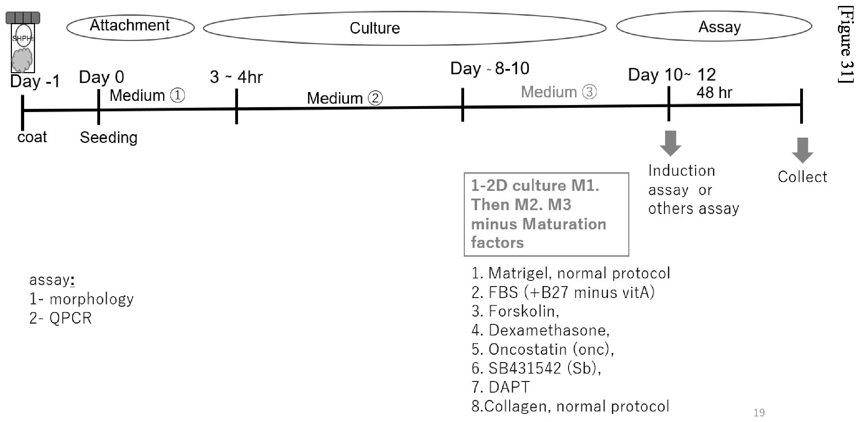

An outline of the experimental protocol of Example 8 is shown in Figure 31. The influence caused by omission of culturing in Medium 3 was examined.

### [Methods]

After coating the wells, the cells were seeded using Medium 1, and 3 to 4 hours later, the cells were cultured in Medium 2 for 8 days, and the cells were then cultured using Medium 2 until Day 10, obtain a morphological photograph.

### [Results]

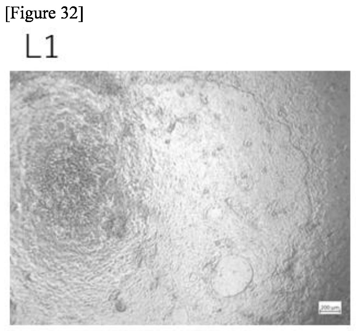

Culturing in Medium 3 enhanced cell function, but the cells adhered and could be cultured in a living state, even without culturing in Medium 3 (Figure 32).

### < Example 9 >

### [Methods]

### 22. Two-dimensional culture system: (A two-dimensional culture system was used to investigate the factors of sHPHs.)

Using an ordinary protocol consisting of culturing in Medium 1, Medium 2, and Medium 3, sHPHs were seeded at 3 x 10⁴ cells/cm² on a matrix two-dimensional Transwell plate pre-coated with Matrigel, as shown in groups below. Briefly, sHPHs were seeded in Medium 1, and were cultured for 3 hours, and were then cultured at 37°C under normal oxygen conditions (5% CO₂ incubator). Next, the medium was changed every day, and the cells were cultured in Medium 2 for about 8 days. Subsequently, in order to improve the maturation function of the cultured cells, the medium was changed, and the cells were cultured for a further 2 days in Medium 3, or in Medium 3 with each factor removed, as shown below. After that, all factors were removed, and a sandwich culture was performed using Matrigel for 1 day. Thereafter, the cells were washed with a phosphate-buffered saline (PBS), and were then prepared for induction assays for evaluating the functions of CYP1A2 and CYP3A4.

1. All factors (ordinary protocol including all steps in Medium 1, Medium 2, and Medium 3). Subsequently, induction assays were performed using DMSO and RIF (rifampicin).
2. Medium 1, Medium 2, and Medium 3. FBS was not used (vitamin A-removed B27 was comprised). Subsequently, induction assays were performed.
3. Medium 1, Medium 2, and Medium 3. Forskolin was not used in Medium 3. Subsequently, induction assays were performed.
4. Medium 1, Medium 2, and Medium 3. Dexamethasone was not used in Medium 3. Subsequently, induction assays were performed.
5. Medium 1, Medium 2, and Medium 3. oncostatin M (onc) was not used in Medium 3. Subsequently, induction assays were performed.
6. Medium 1, Medium 2, and Medium 3. SB431542 (Sb) was not used in Medium 3. Subsequently, induction assays were performed.
7. Medium 1, Medium 2, and Medium 3. DAPT was not used in Medium 3. Subsequently, induction assays were performed.
8. Collagen was used instead of Matrigel, and the ordinary protocol, including all steps, was used. Subsequently, induction assays were performed.

### 23. CYP induction assay

CYP1A2 and CYP3A4 were each induced by a treatment with 50 µM omeprazole (Omep) and 25 µM rifampicin (RIF), respectively. An equal amount of DMSO (0.1%) were used as a vehicle control, and the cells were incubated in an incubator of 5% CO₂ and 37°C for 48 hours. Each CYP induction medium was changed with a freshly prepared medium every day, and the cells were harvested for real-time PCR.

### [Results]

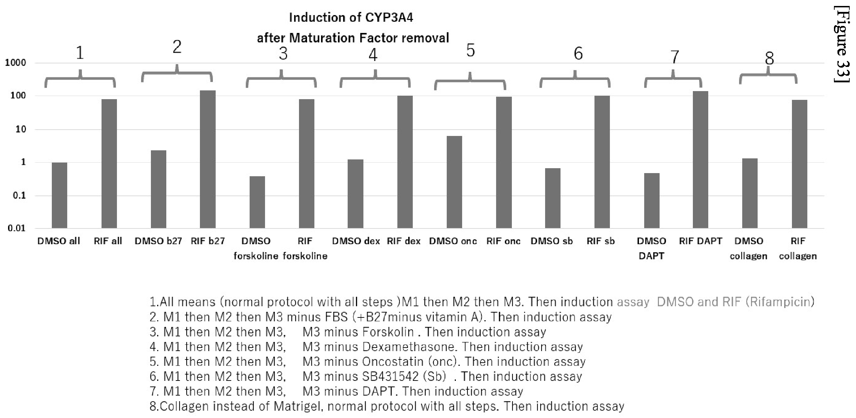

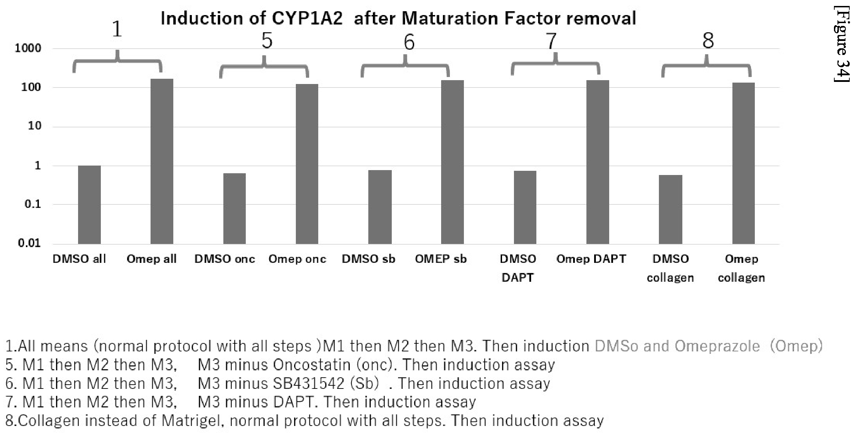

Even if only one factor was removed from Medium 3, it did not affect the induction function of CYP1A2 and CYP3A4 (Figures 33 and 34). In order to investigate the functions of the remaining CYPs after the removal of the Medium 3 factors, and other transporters, further experiments are needed.

### < Conclusion >

1. Even if the factors A83-01 (A) and CHIR99021 (CH) have been removed from the medium, sHPHs can survive. Therefore, the mechanisms of sHPH adhesion, anti-apoptosis, and culture may be different from those of proliferation, and further clarification thereof is needed.
2. Serum or serum replacement components must be FBS, KSR, or B27 (0.5% to 25%), but pHPHs can be cultured even without serum.
3. Multiple factors are necessary for sHPH adhesion and culture, but good morphology was obtained even when one factor was removed from the medium. Thus, further experiments are needed to identify the factors (removing one factor may reveal the existence of another factor with a similar action).
4. From this experiment and the previous experiment, it was found that the components of Medium 1 and Medium 2 could reduce caspase and could promote sHPH adhesion and culture. To such promotion of adhesion and culture, juvenilization (immaturation) of cells due to partial reprogramming is likely to contribute. However, further experiments are needed to clarify the mechanism.
5. The cells can survive without culture in Medium 3, but Medium 3 enhances the maturation function of sHPHs.
6. Examples of usable extracellular matrices (Matrigel, iMatrix-511 laminin 511 (60% of Matrigel components), and collagen).

## Claims

1. A method for producing plateable hepatocytes from suspension hepatocytes, or suspension hepatic cells comprising (ii) culturing suspension hepatocytes in the presence of at least 5 types selected from a ROCK inhibitor, a TGFβ receptor inhibitor, a GSK-3β inhibitor, an epidermal growth factor (EGF), a hepatocyte growth factor (HGF), a sphingophospholipid, and a glycerophospholipid.

2. The production method according to Claim 1, comprising (i) culturing suspension hepatocytes in the presence of at least 4 types selected from a ROCK inhibitor, EGF, HGF, a sphingophospholipid, and a glycerophospholipid, before the culture of (ii).

3. The production method according to Claim 1, wherein the culture of (ii) comprises forming spheroids from suspension hepatocytes.

4. The production method according to Claim 1 or 2, wherein the cultures of (i) and (ii) are carried out on a culture surface coated with a basement membrane component.

5. The production method according to any one of Claim 1 to 3, comprising (iii) culturing the cells in the presence of at least 9 types selected from a ROCK inhibitor, a TGFβ receptor inhibitor, a GSK-3β inhibitor, a cAMP signaling activator, EGF, HGF, a sphingophospholipid, a glycerophospholipid, a γ secretase inhibitor, oncostatin M, and dexamethasone, following the culture of (ii).

6. The production method according to Claim 1, which comprises performing a passage operation after the culture of (ii).

7. The production method according to Claim 1, which comprises cryopreserving the hepatocytes and thawing them, after the culture of (ii).

8. The production method according to Claim 1 or 2, wherein the sphingophospholipid is sphingosine-1-phosphate or a salt thereof, and the glycerophospholipid is lysophosphatidic acid or a salt thereof.

9. The production method according to Claim 1, wherein the ROCK inhibitor is Y27632, the TGFβ receptor inhibitor is A83-01, and the GSK-3β inhibitor is CHIR99021.

10. The production method according to Claim 1, wherein the suspension hepatocytes are suspension primary hepatocytes.

11. The production method according to Claim 1, wherein the suspension hepatocytes are human suspension primary hepatocytes.

12. A method for measuring the metabolism of a test substance, comprising the following steps (1) to (3):
(1) A step of obtaining plateable hepatocytes by the production method according to any one of [1] to [11] above;
(2) A step of allowing a test substance to come into contact with the plateable hepatocytes obtained in the step (1); and
(3) A step of measuring the metabolism of the test substance.

13. A method for inducing a metabolic enzyme to hepatocytes, comprising the following steps (1) and (2):
(1) A step of obtaining plateable hepatocytes by the production method according to any one of [1] to [11] above; and
(2) A step of allowing a metabolic enzyme inducer to come into contact with the plateable hepatocytes obtained in the step (1).

14. The method according to Claim 14, which comprises subjecting the plateable hepatocytes obtained in the step (1) to a sandwich culture.

15. A method for producing hepatocyte spheroids, comprising the following steps (1) and (2):
(1) A step of obtaining plateable hepatocytes by the production method according to any one of Claim 1 to 3; and
(2) A step of culturing the plateable hepatocytes obtained in the step (1) to form hepatocyte spheroids.

16. A method for subjecting hepatocytes to a two-dimensional culture on cell culture inserts, comprising the following steps (1) to (3):
(1) A step of obtaining plateable hepatocytes by the production method according to any one of Claim 1 to 3;
(2) A step of converting the plateable hepatocytes obtained by the step (1) into single cells; and
(3) A step of subjecting the hepatocytes converted into single cells in the step (2) to a two-dimensional culture on cell culture inserts such as Transwell.

17. The method according to Claim 16, wherein the culture in the step (3) is carried out on a culture surface coated with a basement membrane component.

18. A medium used in the method according to Claim 2, comprising at least 4 types selected from a ROCK inhibitor, EGF, HGF, a sphingophospholipid, and a glycerophospholipid.

19. A medium used in the method according to Claim 1, comprising at least 5 types selected from a ROCK inhibitor, a TGFβ receptor inhibitor, a GSK-3β inhibitor, EGF, HGF, a sphingophospholipid, and a glycerophospholipid.

20. A kit, comprising the medium according to Claim 18, the medium according to Claim 19, and suspension hepatocytes.
